(19) Europäisches Patentamt
European Patent Office
Office européen des brevets

(11) **EP 0 781 150 B1**

(12) **EUROPEAN PATENT SPECIFICATION**

(45) Date of publication and mention
of the grant of the patent:
**06.11.2002 Bulletin 2002/45**

(51) Int Cl.⁷: $A61M\ 37/00$, $A61N\ 1/32$

(21) Application number: **96925491.1**

(86) International application number:
**PCT/US96/12244**

(22) Date of filing: **25.07.1996**

(87) International publication number:
**WO 97/004832 (13.02.1997 Gazette 1997/08)**

(54) **ENHANCED TRANSDERMAL TRANSPORT USING ULTRASOUND**

VERBESSERTER TRANSDERMALER TRANSPORT UNTER VERWENDUNG VON ULTRASCHALL

TRANSPORT TRANSDERMIQUE AMELIORE GRACE AUX ULTRASONS

(84) Designated Contracting States:
**AT BE CH DE DK ES FR GB GR IE IT LI LU MC NL PT SE**

(30) Priority: **25.07.1995 US 507060**
**04.08.1995 US 511583**
**18.12.1995 US 574377**
**01.04.1996 US 626021**

(43) Date of publication of application:
**02.07.1997 Bulletin 1997/27**

(73) Proprietor: **MASSACHUSETTS INSTITUTE OF TECHNOLOGY**
**Cambridge, MA 02139 (US)**

(72) Inventors:
• **MITRAGOTRI, Samir, S.**
**Somerville, MA 02138 (US)**
• **PLIQUETT, Uwe**
**D-04105 Leipzig (DE)**
• **JOHNSON, Mark, E.**
**Allston, MA 02134 (US)**
• **PISHKO, Michael**
**Arlington, MA 02174 (US)**
• **KOST, Joseph**
**84965 Omer (IL)**
• **LANGER, Robert, S.**
**Newton, MA 02158 (US)**
• **WEAVER, James, C.**
**Sudbury, MA 01776 (US)**
• **BLANKSCHTEIN, Daniel**
**Brookline, MA 02146 (US)**

(74) Representative: **Stevens, Ian Edward et al**
**Eric Potter Clarkson,**
**Park View House,**
**58 The Ropewalk**
**Nottingham NG1 5DD (GB)**

(56) References cited:
| EP-A- 0 368 408 | EP-A- 0 612 525 |
| WO-A-88/00001 | WO-A-90/01971 |
| US-A- 5 115 805 | US-A- 5 323 769 |
| US-A- 5 386 837 | US-A- 5 415 629 |
| US-A- 5 421 816 | US-A- 5 445 611 |

## Description

## Background of the Invention

The present invention is generally in the area of drug delivery, and is particularly improved methods for transdermal drug delivery and monitoring of analytes.

The United States government has rights in this invention by virtue of National Institutes of Health grant No. GM44884 to Robert Langer and Army Office Grant No. DAAL03-90-G0218 to James C. Weaver.

Transdermal drug delivery (TDD) offers several advantages over traditional delivery methods including injections and oral delivery. When compared to oral delivery, TDD avoids gastrointestinal drug metabolism, reduces first-pass effects, and provides sustained release of drugs for up to seven days, as reported by Elias, *In Percutaneous Absorption: Mechanisms-Methodology-Drag Delivery.,* Bronaugh, R. L., Maibach, H. 1. (Ed), pp 1-12, Marcel Dekker, New York, 1989.

Monitoring of analytes using ultrasound has been suggested as an alternative to the use of more invasive procedures. This is particularly attractive for conditions such as diabetes, which requires frequent determination of the blood glucose levels. For example, U.S. Patent No. 5,458,140 to Eppstein, et al., suggests that ultrasound in combination with chemical enhancers is an alternative method for monitoring of blood glucose, although the only examples demonstrate transfer through membranes having pinholes in them.

The word "transdermal" is used herein as a generic term. However, in actuality, transport of drugs occurs only across the epidermis where the drug is absorbed in the blood capillaries. When compared to injections, TDD eliminates the associated pain and the possibility of infection. Theoretically, the transdermal route of drug administration could be advantageous in the delivery of many therapeutic proteins, because proteins are susceptible to gastrointestinal degradation and exhibit poor gastrointestinal uptake, proteins such as interferons are cleared rapidly from the blood and need to be delivered at a sustained rate in order to maintain their blood concentration at a high value, and transdermal devices are easier to use than injections. Ultrasound has been shown to enhance transdermal transport of low-molecular weight drugs (molecular weight less than 500) across human skin, a phenomenon referred to as sonophoresis (Levy, *J. Clin Invest.* 1989, 83, 2974-2078; Langer, R., In *"Topical Drug Bioavailability, Bioequivalence, and Penetration";* pp. 91-103, Shah V. P., M.H.I., Eds. (Plenum: New York, 1993); Frideman, R. M., *'Interferons: A Primer',* Academic Press, New York, 1981)).

U.S. Patent Nos. 4,309,989 to Fahim and 4,767,402 to Kost, et al., disclose various ways in which ultrasound has been used to achieve transdermal drug delivery. Sonophoresis has been shown to enhance transdermal transport of various drugs. Although a variety of ultrasound conditions have been used for sonophoresis, the most commonly used conditions correspond to the therapeutic ultrasound (frequency in the range of 1 MHz - 3 MHz, and intensity in the range of 0 - 2 W/cm$^2$) (Kost, *In Topical Drug Bioavailability Bioequivalence and Penetration,* pp. 91-103, Maibach, H. I., Shah, V. P. (Ed) Plenum Press, New York, 1993; U.S. Patent No. 4,767,402 to Kost, et al.).

In spite of these advantages, very few drugs and no proteins or peptides are currently administered transdermally for clinical applications because of the low skin permeability to drugs. Application of therapeutic ultrasound does not induce transdermal transport of high-molecular weight proteins. It is a common observation that the typical enhancement induced by therapeutic ultrasound is less than ten-fold. In many cases, no enhancement of transdermal drug transport has been observed upon ultrasound application. This low permeability is attributed to the stratum corneum (SC), the outermost skin layer which consists of flat, dead cells filled with keratin fibers (keratinocytes) surrounded by lipid bilayers. The highly-ordered structure of the lipid bilayers confers an impermeable character to the SC (Flynn, G. L., In *Percutaneous Absorption: Mechanisms-Methodology-Drug Delivery.;* Bronaugh, R. L., Maibach, H. I. (Ed), pages 27-53, Marcel Dekker, New York, 1989). Examples of *in vivo* monitoring of analytes using ultrasound have not been published.

A variety of approaches have been suggested to enhance transdermal transport of drugs. These include: i) use of chemicals to either modify the skin structure or to increase the drug concentration in the transdermal patch (Junginger, et al. In *"Drug Permeation Enhancement";* Hsieh, D.S., Eds., pp. 59-90 (Marcel Dekker, Inc. New York 1994; Burnette, R. R. In *Developmental Issues and Research Initiatives*; Hadgraft J., G., R. H., Eds., Marcel Dekker: 1989; pp. 247-288); ii) applications of electric fields to create transient transport pathways [electroporation] (Prausnitz *Proc. Natl. Acad. Sci.USA* 90, 10504-10508 (1993); Walters, K. A., in *Transdermal Drug Delivery: Developmental Issues* and *Research Initiatives,* Ed. Hadgraft J., Guy, R.H., Marcel Dekker, 1989) or to increase the mobility of charged drugs through the skin [iontophoresis], and iii) application of ultrasound [sonophoresis]. Various approaches including chemical enhancers [Walters, K.A., in *Transdermal Drug Delivery:* Developmental *Issues and Research* Initiatives, Hadgraft J., Guy, R.H., Marcel Dekker: New York (1989)], ultrasound [Levy et al., *J. Clin. Invest.,* 83: 2074-2078 (1989); Mitragotri et al., *J. Pharm. Sci,* 84:697-706 (1995)] and electrical enhancement [Prausnitz et al. *Proc. Natl. Acad.* Sci.USA, 90: 10504-10508 (1993); Pliquett et al., Pharmaceutical Research, 12:549-555 (1995); Chizmadzhev et al., *Biophysical J.* 68:749-765 (1995); Burnette (1989)] have been suggested to enhance transdermal drug transport. Chemical enhancers have been found to increase transdermal drug transport via several different mechanisms, including increased solubility of the drug in the donor formulation, increased partitioning into the SC, fluidization of the lipid bilayers, and disruption of the intracellular proteins (Kost and Langer, In Topical Drug Bioavailability, Bioequivalence, and Penetration; Shah and Maibech, ed. (Plennum, NY 1993) pp. 91-103 (1993)). U.S. Patent No. 5,445,611 to Eppstein, et al., describes enhancement of ultrasound using the combination of chemical enhancers with modulation of the frequency, intensity, and/or phase of the ultrasound to induce a type of pumping action. However, the intensity and frequencies used in the examples are quite high, which generates heat and decreasing transport over time. Electroporation is believed to work in part by creating transient pores in the lipid bilayers of the SC (Burnett (1989)). Iontophoresis provides an electrical driving force to move compounds. Electroporation involves application of electric field pulses that create transient aqueous pathways in lipid bilayer membranes, causing a temporary alteration of skin structure. While occurrence of aqueous pores may allow transdermal permeation of neutral molecules by diffusion, the transport of charged molecules during pulsing occurs predominantly by electrophoresis and electroosmosis. In some cases, high strengths of the physico-chemical forces (for example, electricity, ultrasound) are required to deliver a given drug dose transdermally. However, the highest strength of these physico-chemical forces that can be used is limited by their adverse physiological effects.

US 5421816 discloses the use of ultrasonic energy to release a stored drug and move it through the skin into the blood stream.

US 5323769 teaches the delivery of a drug in conjunction with ultrasound having a frequency of above about 10 MHz.

US 5115805 describes the delivery of a drug using ultrasound having a frequency of above about 10 MHz.

US 5415629 discloses apparatus for iontophoretic or iontophoretic-ultrasonic transdermal delivery of medication across the skin.

EP-A-0612525 teaches transdermal drug delivery systems which have reduced skin irritating or sensitising effects.

EP-A-0368408 describes a storage-stable transdermal patch.

US 5386837 discloses an electrochemotherapeutic method comprising the application of force-field shock pulses.

WO 90/01971 teaches a method for enhancing and controlling transbuccal infusion of molecules using ultrasound.

Accordingly, a better selection of ultrasound parameters is needed to induce a higher enhancement of transdermal drug transport by sonophoresis.

Moreover, although efficacy to some degree has been observed using ultrasound for transport of other compounds, the efficiency of transport under conditions acceptable to patients has not been achieved.

It is therefore an object of the present invention to provide a method and means for enhancing transdermal transport for transdermal drug delivery and monitoring of analyte.

It is a further object of the present invention to provide methods for using ultrasound in combination with other means of enhancement for drug delivery and collection of analyte in an efficient, practical manner.

It is a further object of the present invention to provide an improved, painless method for obtaining a patient sample for measurement of analytes in blood or other body fluids.

## Summary of the Invention

Several means for enhancing transdermal transport of drugs and analytes have been developed, including the use of low frequency ultrasound, chemical modifiers of permeability and/or cavitation, iontophoresis and/or electroporation (electric fields), pressure and/or vacuum (physical enhancers), and magnetic force fields. Applications of low-frequency (approximately 20 KHz to 1 MHz) ultrasound enhances transdermal transport of drugs and measurements of the concentration of analytes in body fluids such as blood or lymph. In a preferred embodiment, the ultrasound is low frequency ultrasound which induces cavitation, thereby increasing the permeability of the stratum corneum. Delivery can be further enhanced or controlled through the use of carriers for the drugs, such as liposomes or microparticles, using a wide range of ultrasound frequency ranges and intensities. The microparticles are preferably small, and may have surfaces with increased hydrophilicity or lipophilicity to further enhance transport.

Transdermal transport of molecules during sonophoresis (delivery or extraction) can be further enhanced by providing chemical enhancers which increase the solubility of the compound to be transported and/or lipid bilayer solubility, or additional driving forces for transport, such as, mechanical force fields, magnetic fields or iontophoresis.

Transdermal transport of molecules during sonophoresis (delivery or extraction) can be further enhanced by the application of an electric field, for example, by iontophoresis or electroporation. Still further enhancement can be obtained using a combination of chemical enhancers and/or magnetic field with the electric field and ultrasound.

Examples using low frequency ultrasound demonstrate *in vitro* and *in vivo* administration of insulin (molecular weight 6,000 D), and *in vitro* administration of gamma interferon (molecular weight 17,000 D), and erythropoeitin (molecular weight 48,000 D). Examples using low frequency ultrasound also demonstrate measurement of blood glucose *in vitro* and *in vivo*. Additional examples compare the effects and mechanisms of (i) a series of chemical enhancers, and (ii) the combination of these enhancers and therapeutic ultrasound (1 MHz, 1.4 W/cm2) on transdermal drug transport. Initial/comprehensive experiments were performed with a model drug, corticosterone, and a series of chemical enhancer formulations, including polyethylene glycol 200 dilaurate (PEG), isopropyl myristate (IM), glycerol trioleate (GT), ethanol/pH 7.4 phosphate buffered saline in a one-to-one ratio (50% ethanol), 50% ethanol saturated with linoleic acid (LA/ethanol), and phosphate buffered saline (PBS). Examples using two model compounds, calcein and sulphorhodamine, demonstrate that transdermal transport enhancement induced by simultaneous application of ultrasound and electric pulses is higher than that due to electric pulses or ultrasound alone. Application of ultrasound reduces the threshold voltage required for the onset of calcein and sulphorhodamine transport in the presence of electric fields.

**Brief Description of the Drawings**

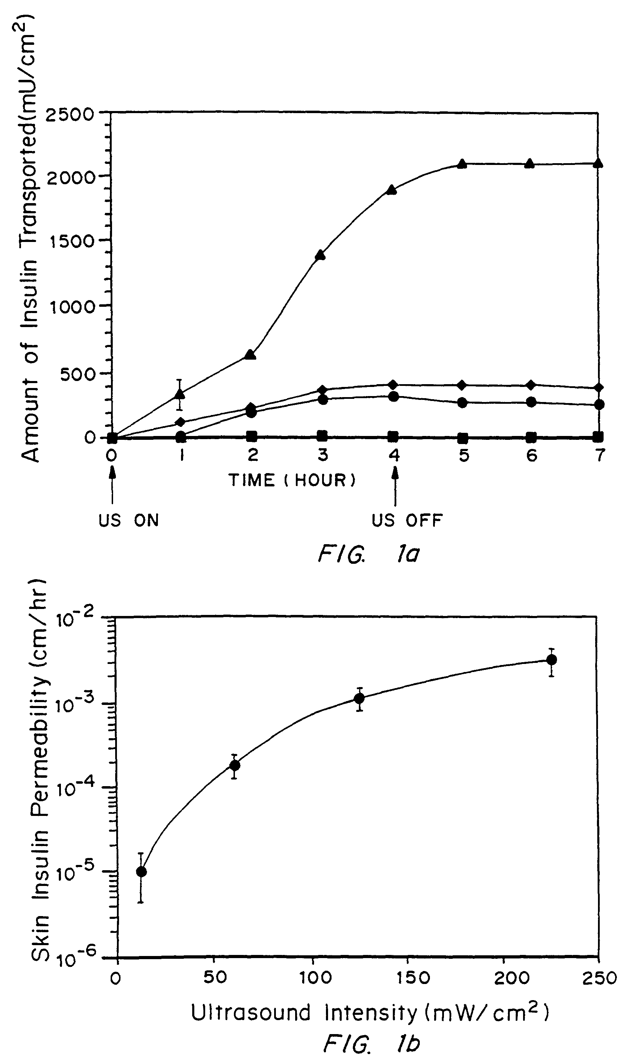

Figure 1a is a graph of the amount of insulin transported across human skin (in *vitro*) in the presence of ultrasound (20 KHz, 100 msec pulses applied every second) at various intensities ($\blacksquare$ - 12.5 mW/cm2, $\blacklozenge$ - 62.5 mW/cm2, $\bullet$ - 125 mW/cm2, and $\blacktriangle$ - 225 mW/cm2). (n=3-4, error bars indicate SD (Standard Deviation))

Figure 1 is a graph of the variation of the transdermal insulin permeability (in *vitro*) with ultrasound intensity (20 KHz, 100 msec pulses applied every second). (n=3-4, error bars indicate SD.) Note that the skin is impermeable to insulin at an ultrasound intensity =O.

Figure 2 is a graph of glucose concentration in the donor compartment (measured as percent of received glucose) over time (minutes) in an *in vitro* system.

Figure 3 is a graph of glucose concentration in the donor compartment (mg/dl) after ten minutes as a function of glucose concentration in the receiver compartment (mg/dl).

Figure 4 is a graph of solubility (mg/ml) for corticosterone, dexamethasone, and testosterone in PBS (dark bar), 50% ethanol (hatched \\\\) and linoleic acid in 50% ethanol (striped ||||).

Figure 5 is graph of the permeability enhancement for testosterone (288.4 Da), corticosterone (346.5 Da), dexamethasone (392.5 Da) in combination with linoleic acid (dark bars) and ultrasound in combination with linoleic acid enhancement (\\\\).

Figure 6a is a graph of enhancement (log scale) versus molecular weight (Da). Figure 6b is a graph of permeability enhancement versus molecular weight (Da).

Figure 7a is a graph of fraction of corticosterone transported (%) versus time (hours). Figure 7b is a graph of the fraction of corticosterone transported (%) versus time (hours).

Figure 8 is a graph of the fraction of corticosterone transported (%) versus time (hours).

Figure 9 is a graph of the amount of calcein transported in one hour (fraction of the amount in the donor x 10^5) for sonophoresis alone, iontophoresis alone, and sonophoresis in combination with iontophoresis.

Figure 10 is a graph of the glucose concentration in the donor (fraction of receiver concentration).

Figure 11a is a graph of sulforhodamine flux/$\mu$g/(cm2h) over time (seconds) for electroporation of sulforhodamine, followed by application of electroporation in combination with ultrasound. After 400 sec of passive diffusion, pulsed ultrasound (1 MHz, 20% duty cycle, 2.5 - 2.9 W/cm2) was turned on for 2750 sec. The ultrasound was turned off at 2750 sec. High voltage pulsing was turned on at 6900 sec for 1 hour (10,500 sec end of electroporation pulsing). Ultrasound (1 MHz, 0.8 cm2 20% duty cycle, 2.5 - 2.9 W/cm2) was turned on again at 14,310 sec, electroporation was turned on again at 15,200 sec while the pulsed ultrasound was on. At 16,440 sec the ultrasound wave was changed from pulsed to continuous while the electroporation continued.

Figure 11b is a graph of the time variation of calcein flux in the presence of electric fields alone (X) and during simultaneous application of ultrasound and electric field (O) (1 MHz, 1.4 W/cm2, continuous application, and electric field, 100 V across the skin, exponentially decaying pulse with a time constant ($\tau$) of 1 millisecond, one pulse applied every minute). Ultrasound was ON all the time (O). Electric voltage was turned ON at time 0 and was turned OFF at 1 hour in both the case (O as well as X). Presented as means and S.D. of at least three repetitions.

Figure 11c. Time variation of sulphorhodamine flux in the presence of electric field alone (X) and during simultaneous application of ultrasound and electric field (O) (1 MHz, 1.4 W/cm2, continuous application, and electric field, 100 V across the skin, exponentially decaying pulse with a time constant ($\tau$) of 1 millisecond, one pulse applied every minute). Ultrasound was ON all the time (O). Electric voltage was turned ON at time O and was turned OFF at 1 hour in both the case (O as well as X). Presented as means and S.D. of at least three repetitions.

Figure 12a and 12b are graphs of calcein and sulphorhodamine flux over time (hours), respectively. Skin samples were exposed continuously to electroporation (electric field (750 V across the chamber, equivalent to approximately 210-230 volts across the skin, exponentially decaying pulse with a time constant ($\tau$) of 1 millisecond, one pulse applied every minute) and continuous ultrasound (1 MHz, 0.8 cm$^2$, 2 W/cm$^2$) (o) and controls (x) where the skin was exposed to electric fields alone.

Figure 13 is a graph of the variation of the transdermal sulphorhodamine flux with the applied electric field (100 V across the skin, exponentially decaying pulse with a time constant ($\tau$) of 1 millisecond, one pulse applied every minute) in the presence (O) and absence (X) of ultrasound. Presented as means and S.D. of at least three repetitions.

Figure 14 is a graph showing the variation of the normalized transdermal calcein and sulphorhodamine flux under a variety of conditions. A- in the presence of electric field alone, B- in the presence of ultrasound and electric field, C-in the presence of ultrasound alone, D- in the absence of ultrasound and electric field. The transdermal calcein and sulphorhodamine fluxes have been normalized by the corresponding fluxes prior to application of ultrasound, that is, at the end of 0.5 hours. This was done to assist comparison of the relative charges in transdermal flux under different conditions.

## Detailed Description of the Invention

### *Sonophoresis:*

As used herein, sonophoresis is the application of ultrasound to the skin, alone or in combination with chemical enhancers, iontophoresis, electroporation, magnetic force fields, mechanical pressure fields or electrical fields, to facilitate transport of a compound through the skin. In one embodiment, a drug, alone or in combination with a carrier, penetration enhancer, lubricant, or other pharmaceutically acceptable agent for application to the skin, is applied to the skin. In another embodiment, the compound is an analyte such as glucose which is present in a body fluid and extracted by application of the ultrasound, alone or in combination with other forces and/or chemical enhancers.

Ultrasound is defined as sound at a frequency of between 20 kHz and 10 MHz, with intensities of between greater than 0 and 3 W/cm$^2$. Ultrasound is preferably administered at frequencies of less than or equal to about 2.5 MHz to induce cavitation of the skin to enhance transport. Exposures are typically for between 1 and 100 minutes, but may be shorter and/or pulsed.

As used herein, "low frequency" sonophoresis is ultrasound at a frequency that is less than 1 MHz, more typically in the range of 20 to 40 KHz, which can be applied continuously or in pulses, for example, 100 msec pulses every second, at intensities in the range of between zero and 1 W/cm$^2$, more typically between 12.5 mW/cm$^2$ and 225 mW/cm$^2$. It should be understood that although the normal range of ultrasound begins at 20 kHz, one could achieve comparable results by varying the frequency to slightly more or less than 20 kHz.

Many ultrasound devices are available commercially which can be used in the method described herein. For example, the ultrasonic devices used by dentists to clean teeth have a frequency of between about 25 and 40 KHz. Commercially available portable ultrasound toothbrushes make use of a small sonicator contained within the toothbrush (Sonex International Corporation). This sonicator is portable and operates on rechargeable batteries. Small pocket-size sonicators carried by patients and used to "inject" drugs whenever required could be readily adapted from these devices. In addition, these devices could be combined with sensors that can monitor drug concentrations in the blood to formulate a self-controlled drug (insulin, for example) delivery method that can decrease the attention required by the patient.

Devices typically used for therapeutic or diagnostic ultrasound operate at a frequency of between 1.6 and 10 MHz. These devices can also be modified for use at lower frequencies. The devices may optionally include a reservoir for an ultrasound gel, which will typically have an acoustic impedance like water, or a reservoir for collecting analyte. Although principally described herein as the combination of ultrasound with an electrical field, chemical enhancers and physical enhancers can also be used in combination with ultrasound. Physical enhancers, as used herein, in addition to iontophoresis and electroporation, include magnetic fields and mechanical pressure or vacuum. Ultrasound is used to permeabilize the skin followed by the application of various force fields to provide additional driving force for transdermal transport of molecules.

### *Chemical Enhancers.*

### Lipid Bilayer Disrupting Agents.

Chemical enhancers have been found to increase drug transport by different mechanisms. Chemicals which enhance permeability through lipids are known and commercially available. For example, ethanol has been found to increase the solubility of drugs up to 10,000-fold and yield a 140-fold flux increase of estradiol, while unsaturated fatty acids have been shown to increase the fluidity of lipid bilayers (Bronaugh and Maibach, editors (Marcel Dekker 1989) pp. 1-12). Examples of fatty acids which disrupt lipid bilayer include linoleic acid, capric acid, lauric acid, and neodecanoic acid, which can be in a solvent such as ethanol or propylene glycol. Evaluation of published permeation data utilizing lipid bilayer disrupting agents agrees very well with the observation of a size dependence of permeation enhancement for lipophilic compounds. The permeation enhancement of three bilayer disrupting compounds, capric acid, lauric acid, and neodecanoic acid, in propylene glycol has been reported by Aungst, et al. *Pharm. Res.* 7, 712-718 (1990). They examined the permeability of four lipophilic compounds, benzoic acid (122 Da), testosterone (288 Da), naloxone (328 Da), and indomethacin (359 Da) through human skin. The permeability enhancement of each enhancer for each drug was calculated according to $\varepsilon_{c/pg}=P_{e/pg}/P_{pg}$, where $P_{e/pg}$ is the drug permeability from the enhancer/propylene glycol formulation and $P_{pg}$ is the permeability from propylene glycol alone.

The primary mechanism by which unsaturated fatty acids, such as linoleic acid, are thought to enhance skin permeabilities is by disordering the intercellular lipid domain. For example, detailed structural studies of unsaturated fatty acids, such as oleic acid, have been performed utilizing differential scanning calorimetry (Barry *J. Controlled Release* 6, 85-97 (1987)) and infrared spectroscopy (Ongpipattanankul, et al., *Pharm. Res.* 8, 350-354 (1991); Mark, et al., *J. Control. Rel.* 12, 67-75 (1990)). Oleic acid was found to disorder the highly ordered SC lipid bilayers, and to possibly form a separate, oil-like phase in the intercellular domain. SC lipid bilayers disordered by unsaturated fatty acids or other bilayer disrupters may be similar in nature to fluid phase lipid bilayers.

A separated oil phase should have properties similar to a bulk oil phase. Much is known about transport in fluid bilayers and bulk oil phases. Specifically, diffusion coefficients in fluid phase, for example, dimyristoylphosphatidylcholine (DMPC) bilayers Clegg and Vaz In "Progress in Protein-Lipid Interactions" Watts, ed. (Elsevier, NY 1985) 173-229; Tocanne, et al., *FEB* 257, 10-16 (1989) and in bulk oil phase Perry, et al., "Perry's Chemical Engineering Handbook" (McGraw-Hill, NY 1984) are greater than those in the SC, and more importantly, they exhibit size dependencies which are considerably weaker than that of SC transport Kasting, et al., In: "Prodrugs: Topical and Ocular Delivery" Sloan, ed. (Marcel Dekker, NY 1992) 117-161; *Potts and Guy, Pharm. Res.* 9, 663-339 (1992); Willschut, et al., *Chemosphere* 30, 1275-1296 (1995). As a result, the diffusion coefficient of a given solute will be greater in a fluid bilayer, such as DMPC, or a bulk oil phase than in the SC. Due to the strong size dependence of SC transport, diffusion in SC lipids is considerably slower for larger compounds, while transport in fluid DMPC bilayers and bulk oil phases is only moderately lower for larger compounds. The difference between the diffusion coefficient in the SC and those in fluid DMPC bilayers or bulk oil phases will be greater for larger solutes, and less for smaller compounds. Therefore, the enhancement ability of a bilayer disordering compound which can transform the SC lipids bilayers into a fluid bilayer phase or add a separate bulk oil phase should exhibit a size dependence, with smaller permeability enhancements for small compounds and larger enhancements for larger compounds.

A comprehensive list of lipid bilayer disrupting agents is described in European Patent Application 43,738 (1982), which is incorporated herein by reference. Exemplary of these compounds are those represented by the formula:

$$R\text{-}X,$$

wherein R is a straight-chain alkyl of about 7 to 16 carbon atoms, a non-terminal alkenyl of about 7 to 22 carbon atoms, or a branched-chain alkyl of from about 13 to 22 carbon atoms, and X is -OH, - $COOCH_3$, - $COOC_2H_5$, - $OCOCH_3$, - $SOCH_3$, - $P(CH_3)_2O$, $COOO_2H_4OC_2H_4OH$, - COOCH (CHOH) $_4CH_2OH$, - $COOCH_2$, $CHOHCH_3$, $COOCH_2CH(OR")$ $CH_2OR"$, -$(OCH_2CH_2)_mOH$, -COOR', or -$CONR'_2$ where R' is - -H, -$CH_3$, -$C_2H_5$, -$C_2H_7$ or -$C_2H_4OH$; R" is -H, or a non-terminal alkenyl of about 7 to 22 carbon atoms; and m is 2-6; provided that when R" is an alkenyl and X is -OH or -COOH, at least one double bond is in the cis-configuration.

**Solubility Enhancers**

Suitable solvents include water; diols, such as propylene glycol and glycerol; mono-alcohols, such as ethanol, propanol, and higher alcohols; DMSO; dimethylformamide; N,N-dimethylacetamide; 2-pyrrolidone; N-(2-hydroxyethyl) pyrrolidone, N-methylpyrrolidone, 1-dodecylazacycloheptan-2-one and other n-substituted-alkyl-azacycloalkyl-2-ones and other n-substituted-alkyl-azacycloalkyl-2-ones (azones).

U.S. Patent No. 4,537,776 to Cooper contains a summary of prior art and background information detailing the use of certain binary systems for permeant enhancement. European Patent Application 43,738, also describes the use of selected diols as solvents along with a broad category of cell-envelope disordering compounds for delivery of lipophilic pharmacologically-active compounds. A binary system for enhancing metaclopramide penetration is disclosed in UK Patent Application GB 2,153,223 A, consisting of a monovalent alcohol ester of a C8-32 aliphatic monocarboxylic acid (unsaturated and/or branched if C18-3 or a C6-24 aliphatic monoalcohol (unsaturated and/or branched if C14-24) and an N-cyclic compound such as 2-pyrrolidone or N-methylpyrrolidone.

Combinations of enhancers consisting of diethylene glycol monoethyl or monomethyl ether with propylene glycol monolaurate and methyl laurate are disclosed in U.S. Patent No. 4, 973,468 for enhancing the transdermal delivery of steroids such as progestogens and estrogens. A dual enhancer consisting of glycerol monolaurate and ethanol for the transdermal delivery of drugs is described in U.S. Patent No. 4,820,720. U.S. Patent No. 5,006,342 lists numerous enhancers for transdermal drug administration consisting of fatty acid esters or fatty alcohol ethers of $C_2$ to $C_4$ alkanediols, where each fatty acid/alcohol portion of the ester/ether is of about 8 to 22 carbon atoms. U.S. Patent No. 4,863,970 discloses penetration-enhancing compositions for topical application including an active permeant contained in a penetration-enhancing vehicle containing specified amounts of one or more cell-envelope disordering compounds such as oleic acid, oleyl alcohol, and glycerol esters of oleic acid; a $C_2$ or $C_3$ alkanol and an inert diluent such as water.

Other chemical enhancers, not necessarily associated with binary systems, include dimethylsulfoxide (DMSO) or aqueous solutions of DMSO such as those described in U.S. Patent No. 3,551,554 to Herschler; U.S. Patent No. 3,711,602 to Herschler; and U.S. Patent No. 3,711,606 to Herschler, and the azones (n-substituted-alkyl-azacycloalkyl-2-ones) such as noted in U.S. Patent No. 4,557,943 to Cooper. Some chemical enhancer systems may possess negative side effects such as toxicity and skin irritations. U.S. Patent No. 4,855,298 discloses compositions for reducing skin irritation caused by chemical enhancer-containing compositions having skin irritation properties with an amount of glycerin sufficient to provide an anti-irritating effect.

## Combinations of Lipid Bilayer Disrupting Agents and Solvents

Passive experiments without ultrasound with polyethylene glycol 200 dilaurate (PEG), isopropyl myristate (IM), and glycerol trioleate (GT) result in corticosterone flux enhancement values of only 2, 5, and 0.8, relative to the passive flux from PBS alone. However, 50% ethanol and LA/ethanol significantly increase corticosterone passive fluxes by factors of 46 and 900. These passive flux enhancements were due to (1) the increased corticosterone solubility in the enhancers, and (2) interactions of linoleic acid with the skin. Specifically, linoleic acid increased the corticosterone permeability by nearly 20-fold over that from 50% ethanol alone. Therapeutic ultrasound (1 MHz, 1.4 W/cm$^2$) and the chemical enhancers utilized together produce corticosterone fluxes from PBS, PEG, IM, and GT that are greater than the passive fluxes from the same enhancers by factors of between 1.3 and 5.0, indicating that the beneficial effects of chemical enhancers and therapeutic ultrasound can be effectively combined. Ultrasound combined with 50% ethanol produces a 2-fold increase in corticosterone transport above the passive case, but increase by 14-fold the transport from LA/Ethanol. The combination of increased corticosterone solubility in and permeability enhancement from LA/ethanol and ultrasound yields a flux of 0.16 mg/cm$^2$/hr, 13,000-fold greater than that from PBS alone. The permeability enhancement resulting from the addition of linoleic acid to 50% ethanol exhibits a clear size dependence, with the degree of enhancement increasing with the size of the drug. The degree of permeation enhancement achieved by adding linoleic acid to 50% ethanol and applying ultrasound exhibits a similar size dependence. Ultrasound combined with 50% ethanol produced a 2-fold increase in corticosterone transport above the passive case, but increased by 14-fold the transport from LA/Ethanol. The combination of increased corticosterone solubility in and permeability enhancement from LA/ethanol and ultrasound yields a flux of 0.16 mg/cm$^2$/hr, 13,000-fold greater than that from PBS alone. In order to assess the generality of enhancement ability of LA/ethanol and ultrasound, further experiments were performed with two additional model drugs, dexamethasone and testosterone. As with corticosterone, the solubilities in and passive permeabilities from LA/ethanol were much larger than those from PBS alone for dexamethasone and testosterone. The sonophoretic permeabilities from LA/ethanol were also greater for these two drugs than the passive permeabilities. Moreover, the permeability enhancements of the three drugs resulting from the addition of linoleic acid to 50% 3thanol exhibited a clear size dependence, with the degree of enhancement increasing with the size of the drug. The degree of permeation enhancement achieved by adding linoleic acid to 50% ethanol and applying ultrasound exhibits a similar size dependence. These results suggest that linoleic acid and therapeutic ultrasound, which are both lipid bilayer disordering agents, shift the transport of lipophilic molecules from the passive regime to a regime with a very weak size dependence.

### *Mechanical Forces.*

## Mechanical or Osmotic Pressure

The advantages of combining sonophoresis with physical enhancers is not restricted to electrical current. Effects on transdermal transport may also be observed between ultrasound and pressure (mechanical or osmotic) as well as between ultrasound and magnetic fields since the physical principles underlying the enhancement are believed to be similar or the same. A pressure gradient can be used to enhance convection (physical movement of liquid) across the skin permeabilized by sonophoresis. This can be particularly useful in transdermal extraction of blood analytes. Application of pressure, for example, a vacuum or mechanical pressure, to the skin pretreated by sonophoresis can result in transdermal extraction of interstitial fluid which can be analyzed to measure concentration of various blood analytes.

*Electric Fields (Iontophoresis or Electroporation)*

Application of ultrasound or electric current alone has been shown to enhance transdermal drug transport and blood analyte extraction. Ultrasound-induced cavitation occurring inside or outside the skin causes enhanced transport.

Application of electric current enhances transdermal transport by different mechanisms. First, application of an electric field provides an additional driving force for the transport of charged molecules across the skin and second, ionic motion due to application of electric fields may induce convective flows across the skin, referred to as electroosmosis. This mechanism is believed to play a dominant role in transdermal transport of neutral molecules during iontophoresis. Iontophoresis involves the application of an electrical current, preferably DC, or AC, at a current density of greater than zero up to about 1 mA/cm$^2$.

Attempts have been made to enhance the skin permeability using electric current to achieve transdermal extraction of glucose, as reported by Tamada, et al., *Proceed. Intern. Symp. Control. Rel. Bioact. Mater.* 22, 129-130 (1995). Although these attempts have been successful to a certain extent, the amounts of glucose extracted by these methods are several orders of magnitude lower than those which could be detected by the currently existing biosensors. The mechanism of sonophoretic transdermal glucose extraction is believed to be similar to that of sonophoretic transdermal drug delivery.

In view of this, the cumulative effect of ultrasound and electric field may also be related to cavitation induced by ultrasound exposure. In order to test this hypothesis, electric pulses (100 V across the skin, 1 ms exponential pulse applied every minute) and ultrasound (*3 MHz,* 1.5 W/cm$^2$) were simultaneously applied to skin, as described below. It is known that the cavitational effects vary inversely with ultrasound frequency [Gaertner, W., Frequency dependence of ultrasonic cavitation, *J. Acoust. Soc. Am.,* 26:977-80 (1984)]. No significant cavitational effects have been observed in fluids at high ultrasound frequencies greater than 2.5 MHz. As a result, 2.5 MHz is considered a reasonable estimate of the upper frequency limit for the occurrence of cavitation in fluids at therapeutic ultrasound intensities. Hence, if cavitation plays an important role, the synergistic effect of ultrasound and electric field should be nearly absent when 3 MHz ultrasound is used. Exposure to ultrasound at 3 MHz (intensity = 1.5 W/cm$^2$) does not affect transdermal transport by electric field pulsing. These results indicate that cavitation may play a major role in the synergistic effect of ultrasound and electric field pulsing.

The combination of sonophoresis with an electric field, and optionally, any of these additional physical mechanisms for enhanced transport provides the following advantages over sonophoresis or the physical enhancers alone: i) It allows lowering application times to deliver a given drug dose or extract a certain amount of analytes compared to the required times in the presence of ultrasound or one of the other enhancers alone; ii) It reduces the magnitude of the required ultrasound intensity and electric current or pressure to achieve a given transdermal flux compared to that required if, the enhancers were used alone; and iii) It can be used to provide a better control over transdermal transport of molecules compared to that obtained using an enhancer alone.

The combination of electrical field and ultrasound can be applied to any membrane. The membrane can be skin, cell membrane, cell wall and other biological as well as synthetic membranes. The electric fields can be continuous, pulsed, having high as well as low voltage. Application of ultrasound together with the electrical fields results in higher flux compared to the flux observed with electroporation or ultrasound alone. The onset time of transdermal flux during electroporation can also be reduced by simultaneous applications of ultrasound and electroporation. The effect is more pronounced on less-charged molecules which by other enhancing methods are hard to enhance (iontophoresis). The major limitation of electroporation are the high voltages required in order to cause significant effect. By using the combined effects of ultrasound and electroporation, the intensity levels of the electrical fields will be much lower and therefore no or less damage to the membranes will be observed.

*Magnetic Fields*

Application of magnetic fields to the skin pretreated or in combination with ultrasound may also result in a higher transport of magnetically active species across the skin. For example, polymer microspheres loaded with magnetic particles could be transported across the skin using sonophoresis and magnetic fields.

**Drug Delivery and Monitoring of Analytes**

*Drugs to be Administered.*

Drugs to be administered include a variety of bioactive agents, but are preferably proteins or peptides. Specific examples include insulin, erythropoietin, and interferon. Other materials, including nucleic acid molecules such as antisense and genes encoding therapeutic proteins, synthetic organic and inorganic molecules including antiinflammatories, antivirals, antifungals, antibiotics, local anesthetics, and saccharides, can also be administered.

The drug will typically be administered in an appropriate pharmaceutically acceptable carrier having an acoustic impedance similar to water, such as an aqueous gel. Alternatively, a transdermal patch such as the one described in the examples can be used as a carrier. Drug can be administered in a gel, ointment, lotion, suspension or patch, which can incorporate anyone of the foregoing.

Drug can also be encapsulated in a delivery device such as a liposome or polymeric nanoparticles, microparticle, microcapsule, or microspheres (referred to collectively as microparticles unless otherwise stated). A number of suitable devices are known, including microparticles made of synthetic polymers such as polyhydroxy acids such as polylactic acid, polyglycolic acid and copolymers thereof, polyorthoesters, polyanhydrides, and polyphosphazenes, and natural polymers such as collagen, polyamino acids, albumin and other proteins, alginate and other polysaccharides, and combinations thereof. The microparticles can have diameters of between 0.001 and 100 microns, although a diameter of less than 10 microns is preferred. The microparticles can be coated or formed of materials enhancing penetration, such as lipophilic materials or hydrophilic molecules, for example, polyalkylene oxide polymers and conjugates, such as polyethylene glycol. Liposome are also commercially available.

### *Administration of Drug.*

The drug is preferably administered to the skin at a site selected based on convenience to the patient as well as maximum drug penetration. For example, the arm, thigh, or stomach represent areas of relatively thin skin and high surface area, while the hands and feet are uneven and calloused. In the preferred embodiment, drug is applied to the site and ultrasound and electrical current applied immediately thereafter. Other enhancers can be applied before, during or immediately after the ultrasound. Chemical enhancers are preferable administered during or before ultrasound.

Based on these calculations and experimental data, one can calculate the required dosage and application regime for treatment of a patient, as follows. A typical diabetic patient (70 Kg weight) takes about 12 Units of insulin three times a day (total dose of about 36 Units per day: cited in 'World Book of Diabetes in Practice' Krall, L.P. (Ed), Elsevier, 1988). If each insulin dose was to be delivered by sonophoresis in 1 hour, the required transdermal flux would be 12 U/hour. Note that 1 unit (1 U) of insulin corresponds approximately to 40 $\mu$g of insulin. The transdermal patch area used in these calculations is 40 $cm^2$ (the area of a transdermal FENTANYL™ patch [ALZA Corporation]). The donor concentrations used in these calculations are 100 U/ml in the case of insulin (commercially available insulin solution [Humulin]), 3 x $10^7$ in the case of $\gamma$-interferon (typical concentration of interferon solution recommended by Genzyme Corporation), and 3 x $10^5$ U/ml in the case of erythropoietin [Davis, et al., *Biochemistry,* 2633-2638, 1987].

A typical $\gamma$-interferon dose given each time to patients suffering from cancer or viral infections is about 5 x $10^6$ U [(i) Grups, et al., *Br. J. Med.,* 1989, 64 (3): 218-220, (ii) Parkin, et al., *Br. Med. J.,* 1987, 294: 1185-1186]. Similar doses of $\alpha$-interferon and $\beta$-interferon have also been shown to enhance the immune response of patients suffering from viral infections and cancer (cited in 'Clinical Applications of interferons and their inducers', Ed. Stringfellow D., Marcel Dekker, New York, 1986). If this interferon dose was to be given by sonophoresis in 1 hour, the required transdermal flux would be 5 x $10^6$ U/hour. Note that 1 unit of $\gamma$-interferon corresponds approximately to 1 pg of $\gamma$-interferon.

A typical daily erythropoietin dose given subcutaneously to anemic patients is about 400 U (cited in 'Subcutaneous Erythropoietin, Bommer J., Ritz E., Weinreich T., Bommer G., Ziegler T., Lancet, 406, 1988). If this dose was to be delivered in three steps, each involving sonophoresis for 1 hour, the transdermal flux required would be about 140 U/hour. Note that 1 unit of erythropoietin corresponds approximately to 7.6 nanograms of erythropoietin.

Optimal selection of ultrasound parameters, such as frequency, pulse length, intensity, as well as of non-ultrasonic parameters, such as ultrasound coupling medium, can be conducted to ensure a safe and efficacious application using the guidelines disclosed herein as applied by one of ordinary skill in the art.

### *Analytes to be Measured.*

A variety of analytes are routinely measured in the blood, lymph or other body fluids. Measurements usually require making a puncture in order to withdraw sample. Examples of typical analytes that can be measured include blood sugar (glucose), cholesterol, bilirubin, creatine, various metabolic enzymes, hemoglobin, heparin, vitamin K or other clotting factors, uric acid, carcinoembryonic antigen or other tumor antigens, and various reproductive hormones such as those associated with ovulation or pregnancy.

Transdermal drug delivery, in combination with the non-invasive blood analyte measurements, may be used to formulate self-regulated drug delivery methods which provide a close control of the blood concentrations, minimal pain, and better patient compliance. Non-invasive blood analysis method includes extraction of various analytes from the skin's interstitial fluids (where the analytes are present at a concentration proportional to the blood concentration) across the skin into a patch, solution or gel, where their concentration can be measured using biosensors. This method of blood analyte measurements should be particularly useful in the case of diabetic patients who require multiple daily blood glucose measurements.

### *Measurement of Analytes.*

The ultrasound is applied to the skin at the site where the sample is to be collected. A reservoir or collecting container is applied to the site for collection of the sample, which is then measured using standard techniques. The ultrasound conditions are optimized as in the case for drug delivery, to maximize analyte recovery, while maintaining the relative levels of the analyte to other components of the sample. Chemical and/or physical enhancers are applied to the site before, during and after the ultrasound, preferably during or before the ultrasound.

The present invention will be further understood by reference to the following nonlimiting examples.

### EXAMPLE 1: *In vitro* administration of insulin.

Materials and methods: *In vivo* as well as *in vitro* experiments were performed to study the effect of low-frequency ultrasound on the transport of insulin across the skin. *In vitro* experiments were performed using human cadaver skin. The skin was heat stripped by keeping it in water at 60°C for two minutes followed by the removal of the epidermis. It was then stored at 4°C in a humidified chamber. A piece of epidermis was taken out from the chamber prior to the experiments and was mounted on the Franz diffusion cell (Crown Bioscientific Co.) which consists of two compartments, the donor and the receiver compartment. The human cadaver epidermis (separated from the dermis by heat-treatment) is mounted between the two compartments and is supported by a Nylon mesh (Tetko Inc.) to avoid any damage. The skin was supported by a nylon mesh (Tetko Inc.) in order to a mimic the fact the skin *in vivo* is supported by mechanically strong dermis. The compartments were then clamped together. The receiver compartment was filled with 2% BSA (Sigma Chemicals) solution in PBS (Sigma Chemicals) and the donor solution was filled with 100 U/ml solution of human recombinant insulin (Humulin Regular). The ultrasound intensity, *I*, (Spatial Average Temporal Peak) was calculated from the values of the acoustic pressure amplitude, *P*, measured using a hydrophone (Bruel and Kjaer) using the equation, $I = P^2 / 2\rho c$, where $\rho$ is the water density (1 gm/ml), and c is the velocity of ultrasound in water [1500 m/s].

Ultrasound was turned ON at a frequency of 20 KHz, an intensity varying in the range of 0 to 1 W/cm$^2$ and 10% duty cycle. Samples (200 $\mu$l) were taken from the receiver compartment every hour to measure the concentration of insulin in the receiver compartment. The samples were immediately frozen and were stored at -20° C till they were analyzed by RIA (Linco Research Co.). Ultrasound was typically applied for 4 hours and was then turned OFF. Transdermal insulin flux was followed for next two hours.

Results: The results are shown in Figures 1A and 1B and demonstrate that substantially greater transfer of protein through the skin occurs in the presence of ultrasound. Figures 1a and 1b show the variation of transdermal insulin flux across the human skin *in vitro.* Ultrasound (20 KHz, 125 mW/cm$^2$, 10%) was turned ON at time zero. The insulin flux increased from below the detection limit to a value of about 100 mU/cm$^2$/hr in about 1 hour and stayed almost constant around that value as long as ultrasound was ON. Upon turning ultrasound OFF, the insulin flux decreases and achieves a value below our detection limit within 2 hours after turning ultrasound OFF. The skin permeabilities to insulin at various ultrasound intensities were calculated from the amount of insulin transported during the first hour of ultrasound exposure and are shown in Figure 1b. The sonophoretic permeability varies nearly exponentially with ultrasound intensity, probably due to a highly non-linear dependence of cavitation on ultrasound intensity (Apfel, R. E., *IEEE Trans. Ultrason. Ferroelectrics Freq. Control* 1986, UFFC-33, 139).

Application of ultrasound under these conditions did not appear to cause any permanent loss of the barrier properties of the skin. The transdermal insulin flux (proportional to the slope of the curves shown in Figure 1a) three hours after turning ultrasound OFF was statistically insignificant. To further assess the recovery of the skin barrier properties after sonophoresis, water transport was measured through the skin *during* and *after* ultrasound exposure (20 KHz, 125 mW/cm$^2$, 100 msec pulses applied every second). Transdermal water transport was measured using the same set-up utilized in the insulin experiments, except that the donor compartment was filled with a 1 $\mu$Ci/ml solution of radiolabelled water ($^3$H). The concentration of water in the receiver compartment was measured using, a scintillation counter. During sonophoresis, a water permeability enhancement of 100-fold was observed, of which about 94 ($\pm$ 3) % was recovered within 2 hours after turning ultrasound OFF and 98 ($\pm$ 1)% was recovered within 15 hours. These results suggest that application of ultrasound does not induce any long-lasting loss of the skin barrier properties.

With a transdermal insulin flux of 100 mU/cm$^2$/hr, it should be possible to deliver therapeutic doses of insulin transdermally. Specifically, an insulin dose of about 13 U/h (a dose comparable to the estimated dose required by a diabetic patient if insulin is administered at a controlled rate) could be delivered from a patch having an area of 100 cm$^2$. Accordingly, ultrasound intensity should be useful to control transdermal insulin delivery.

**EXAMPLE 2: In vitro transfer of other proteins.**

Methods and materials: The passive skin permeability to high-molecular weight proteins, including those mentioned above, *is essentially zero* (below the detection limit). To assess whether application of ultrasound enhances transdermal protein flux, the skin permeability to these proteins in the presence of ultrasound *in vitro* across human cadaver epidermis in a Franz Diffusion Cell (Crown Glass Company) was measured. In separate experiments, the donor compartment of the diffusion cell was filled with a solution of insulin (100 U/ml, Humulin Regular, Eli Lilly), γ-interferon (2500 U/ml, Genzyme Corp.), or erythropoeitin (400 U/ml, Amgen Corp.). Ultrasound (20 KHz, 100 msec pulses applied every second) was applied at intensities in the range of 12.5 mW/cm$^2$-225 mW/cm$^2$ for 4 hours using an ultrasound transducer (VCX 400, Sonics and Materials) which was immersed in the donor solution. The transducer having an area of about 1 cm$^2$ was oriented perpendicular to the skin and placed at a distance of 1 cm from the skin. The concentration of proteins in the receiver compartment was measured every hour either by RIA or ELISA. The insulin concentration in the receiver compartment was measured every hour by Radioimmuno Assay (performed at Linco Research Inc., St. Charles). The γ--interferon concentration was measured using ELISA methods developed by Endogen Inc, and the erythropoeitin concentration was measured by ELISA (performed at ARUP, Salt Lake City). Skin permeabilities to proteins were calculated using the transdermal fluxes measured during the first hour. The transdermal flux can be calculated using the equation, $J = DM/\Delta t$, where $\Delta M$ is the amount of protein transported per unit skin area during time $\Delta t$. The skin permeabilities, $P$, can be calculated from the transdermal flux, $J$, during the first hour of ultrasound application using the equation, $P = J/\Delta C$, where $\Delta C$ is the concentration difference across the skin.

Results: Ultrasound application induces significant transdermal permeation of insulin, γ-interferon, and erythropoeitin. As demonstrated in Example 1, the human skin permeability at an ultrasound intensity of 225 mW/cm$^2$ is $3.3 \times 10^{-3}$ ($\pm$ 35%) cm/h to insulin. The permeability to γ-interferon under similar ultrasound conditions is $8 \times 10^{-4}$ ($\pm$ 22%) cm/h, and that to erythropoeitin is $9.8 \times 10^{-6}$ ($\pm$ 40%) cm/h. With these skin permeabilities, it should be possible to deliver these proteins transdermally at a therapeutically relevant rate. The ability of sonophoresis to deliver other macromolecules may be estimated based on their sonophoretic skin permeability which needs to be measured experimentally (generally decreases with increasing molecular size) and the required therapeutic dose of these macromolecules.

**EXAMPLE 3: Transdermal glucose extraction by sonophoresis *in vitro*.**

Application of low-frequency ultrasound can be used to extract glucose across the skin, thus making non-invasive transdermal blood glucose monitoring potentially feasible.

Materials and Methods:

*In Vitro Transdermal Transport Measurements:*

Transdermal transport of a $^{14}$C labeled (New England Nuclear) as well as non-labeled (Sigma Chemicals) was studied in the presence as well as in the absence of low-frequency ultrasound. The permeability experiments were performed *in vitro* using human cadaver skin obtained from local hospitals. The skin was heat stripped by keeping the full-thickness skin in water at 60°C for two minutes followed by the removal of the epidermis. The skin was then stored at 4°C in a humidified chamber for up to 2 weeks. A piece of the epidermis was taken out from the chamber prior to the experiments and was mounted on a Franz diffusion cell (Crown Glass Co., FDC 200). The Franz diffusion cell consists of two compartments, the donor and the receiver compartments, with the stratum comeum facing the donor compartment. The skin was supported by a nylon mesh (Tetko, Inc.) to avoid any damage due to possible mechanical oscillations upon ultrasound application. The donor and receiver compartments were then clamped. The receiver compartment was filled Phosphate Buffer Saline (PBS, phosphate concentration = 0.01 M, NaCl concentration = 0.137M) (Sigma Chemicals Co.) The donor compartment was filled with a solution of either radiolabelled glucose (1 mCi/ml) or non-labeled glucose (concentration in the range of 50 mg/dL to 300 mg/dL) in separate experiments. The concentration of the permeant in the receiver compartment was measured every 5 minutes using a scintillation counter (model 2000 CA, Packard) in the case of radiolabelled glucose and using a commercially available kit (Sigma Chemicals) in the case of unlabeled glucose.

Ultrasound was applied using a sonicator (VCX 400, Sonics and Materials) operating at a frequency of 20 KHz. The ultrasound intensity was measured using a hydrophone (Model 8106, Bruel and Kjaer).

RESULTS

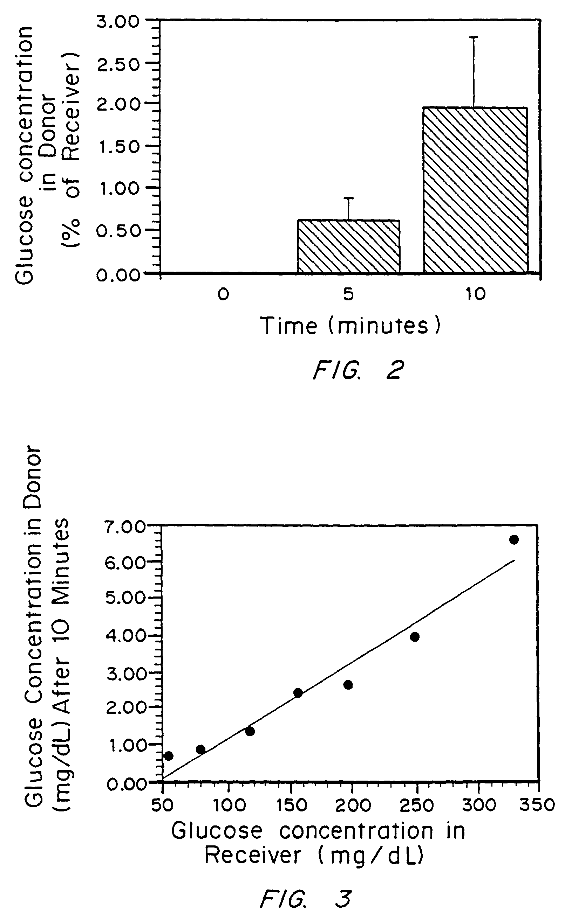

Figure 2 shows the glucose concentration in the donor compartment (represented as percent of the glucose concentration in the receiver compartment) attained at different times during transdermal glucose extraction experiment. The figure shows that even a 5 minute ultrasound application (20 KHz, 125 mW/cm$^2$, continuous) results in a significant glucose transport across human skin *in vitro.* Specifically, the glucose concentration in the donor compartment after 5 minutes of sonophoresis is about 0.5% of that in the receiver compartment. After 10 minutes, the glucose concentration in the donor compartment was about 2% of that in the receiver compartment. The glucose concentration in this range can be measured *in situ* using glucose sensing electrodes, and can be calibrated to indicate actual blood glucose levels.

The amount of glucose extracted by sonophoresis under a given condition varies in the case of skin obtained from different donors (typical variation 40%(SD). However, the variation in the case of skin obtained from the same donor is only about 13%, thus indicating that it should be possible to achieve reliable estimates of glucose concentrations based on transdermal glucose extraction after performing calibration *in vivo* on the patient's skin.

Additional experiments were performed to assess whether the amount of glucose transported by sonophoresis is proportional to the glucose concentration in the receiver compartment. In separate experiments, glucose concentration in the receiver solution was varied from 50 mg/dL to 350 mg/dL (typical variation in the blood glucose level of a diabetic patient) and performed sonophoresis using ultrasound (20 KHz, 125 mW/cm$^2$, continuous) for 10 minutes. Figure 3 shows that the glucose concentration attained in the donor compartment 10 minutes after sonophoresis (represented as percentage of the glucose concentration in the receiver compartment) increased from 0.5 mg/dL to 6.5 mg/dL as glucose concentration in the receiver compartment increased from 50 mg/dL to 350 mg/dL. The line shown in Figure 3 represents the best fit. These results show that the amount of glucose extracted across human skin is proportional to the glucose concentration under the skin, thus indicating that transdermal glucose extraction by sonophoresis could be potentially used for blood glucose measurement.

**EXAMPLE 4: Effect of Ultrasound Intensity and Chemical Enhancers on Transdermal Transport.**

**Materials**

Human cadaver skin from the chest, back, and abdominal regions was obtained from local hospitals and the National Disease Research Institute. The skin was stored at -80°C until usage. The epidermis was separated from the full-thickness tissue after immersion in 60°C water for 2 minutes. Heat-stripped skin was stored at 5°C and 95% humidity for up to 1 week prior to usage. $^3$H-corticosterone, $^3$H-dexamethasone, $^3$H-testosterone, and $^{14}$C-linoleic acid were obtained from DuPont, New England Nuclear. Non-radiolabeled corticosterone (95%), dexamethasone (99+%), testosterone (99+%), and linoleic acid (99%) were obtained from Sigma Chemical. Glycerol trioleate (99+%) and Polyethylene glycol 200 Dilaurate (99+%) were obtained from Henkel. Isopropyl myristate (98%) was obtained from Aldrich Chemicals and butanediol (98%) was obtained from ISP Technologies. Ethanol was obtained from Pharmco Products.

**A. Methods for Passive permeability experiments**

The passive permeabilities (e.g., permeability without the application of ultrasound) of corticosterone, dexamethasone, and testosterone through human skin were measured using trace quantities of radiolabelled drug. The radiolabelled drugs were rotary evaporated in order to remove the ethanol in which they were shipped and any tritium which had reverse exchanged onto it. The radiolabelled drugs were then redissolved in an enhancer formulation to a typical concentration of 1 μCi/ml, and added to the donor chamber of the permeation cell. Passive permeation experiments were performed using stirred side-by-side diffusion cells (Crown Glass, #DC-100B). The receiver compartment always contained pH 7.4 phosphate buffer saline (PBS, phosphate concentration = 0.01 M, NaCl concentration = 0.137 M) (Sigma Chemical Co.). The concentrations of radiolabelled drug in the donor and receiver compartments were measured using a scintillation counter (model 2000 CA, Packard Instruments). A minimum of three experiments were performed with each enhancer formulation.

The permeability values were calculated under steady-state conditions from the relationship $P = (dN_r/dt)/(AC_d)$ *where* A is the surface area of the skin sample, $C_d$ is the drug concentration in the donor chamber, and $N_r$ is the cumulative amount of drug which has permeated into the receptor chamber. The experimentally observed lag-times for the permeation experiments were 1 to 6 hours for corticosterone, 2 to 8 hours for dexamethasone, and less than 1 hour for testosterone. The variability of the individual permeability values were consistent with previously established inter-subject variability of the human skin permeability of 40%, as reported by Williams, et al., *Int. J. Pharm.* 86, 69-77 (1992). The passive permeability enhancements, $E_p$, were calculated relative to the passive permeability from PBS according to Eq. (1):

$$\varepsilon_\rho \equiv \frac{P(enhancer)}{P(PBS)} \tag{1}$$

where *P(enhancer)* is the drug permeability from a given enhancer, and *P(PBS)* is the drug permeability from PBS. The fluxes from saturated solutions, $J^{sat}$, were calculated from $J^{sat}=PC^{sat}$, where $C^{sat}$ is the drug solubility in the formulation. Flux enhancements, $E_j$, were calculated using Eq. (2),

$$\varepsilon_J \equiv \frac{J(enhancer)}{J(PBS)} \qquad (2)$$

where $J^{sat}(enhancer)$ and $J^{sat}(PBS)$ are the drug fluxes from saturated solutions of enhancer and PBS, respectively.

The results of the passive corticosterone transport experiments are shown in Table 1.

**TABLE 1: Corticosterone Transport Properties With Chemical Enhancers**

| Enhancer | Steady-State Permeability, $P$ (cm/hr x10$^5$) | Permeability Enhancement, $\varepsilon_p$ | Solubility, $C^{sat}$ (mg/ml) | Saturated Flux, $J^{sat}$ (mg/cm$^2$/hr x10$^5$) | Flux Enhancement, $\varepsilon_j$ |
|---|---|---|---|---|---|
| Phosphate buffer | 10±32% | 1.0 | 0.12 | 1.2 | 1.0 |
| PEG 200 Dilaurate | 2.4±29% | 0.24 | 0.94 | 2.2 | 1.9 |
| Isopropyl Myristate | 7.0±38% | 0.70 | 0.77 | 5.4 | 4.5 |
| Glycerol trioleate | 7.1±29% | 0.71 | 0.14 | 1.0 | 0.8 |
| 50% Ethanol 50% Buffer | 5.2±21% | 0.52 | 9.2 | 48 | 40 |
| Linoleic acid in 1:1 Ethanol:Buffer | 87±34% | 8.7 | 12.4 | 1080 | 903 |

The results reveal that the enhancer formulations fall into two groups. The first group of chemical enhancers, PEG, IM, and GT, produced only modest effects upon corticosterone transport, while the second group, 50% Ethanol and LA/Ethanol, had a significant impact. With respect to the first group, PEG and IM are better solubilizers of corticosterone, with measured solubilities of 0.94 and 0.77 mg/ml. The solubility of corticosterone in PBS is considerably lower, 0.12 mg/ml, but is similar to the solubility of corticosterone in GT, 0.14 mg/ml, as shown in Table 1. These increases in solubility for PEG and IM, however, do not translate into significantly greater saturated fluxes than that from PBS. This is due to the decreases in the corticosterone permeabilities from PEG and IM relative to that from PBS. Specifically, the measured corticosterone permeability from PBS is $1.0 \times 10^{-4}$ cm/hr, while those from PEG and IM are only $2.4 \times 10^{-5}$ and $7.0 \times 10^{-5}$ cm/hr, as shown in Table 1. As a result, the flux enhancements from PEG and IM are moderate, 1.9 and 4.5, respectively. GT, whose corticosterone solubility is similar to that of PBS, also has a corticosterone permeability, $7.1 \times 10^{-5}$ cm/hr, which is similar to that of PBS, $1.0 \times 10^{-4}$ cm/hr, as shown in Table 1. Thus, the corticosterone flux from a saturated solution of GT, $1.0 \times 10^{-5}$ mg/cm$^2$/hr, is similar to that from saturated PBS, $1.2 \times 10^{-5}$ mg/cm$^2$/hr. In summary, the differences in the solubilities, permeabilities, and fluxes of corticosterone from PBS, PEG, IM, and GT are all relatively moderate.

In contrast, 50% Ethanol and LA/Ethanol significantly increase the transdermal transport of corticosterone. The permeability of corticosterone from 50% Ethanol, $5.2 \times 10^{-5}$ cm/hr, is nearly two-fold lower than that from PBS, $1.0 \times 10^{-4}$ cm/hr, and in the same range as those from PEG, IM, and GT as well. However, 50% Ethanol is a very effective solubilizer. 9.2 mg/ml is the corticosterone solubility in 50% Ethanol, which is nearly 100-fold greater than that in PBS, 0.12 mg/ml, as shown in Table 1. This greater degree of solubilization results in a significantly greater flux of $4.8 \times 10^{-4}$ mg/cm$^2$/hr, which is a factor of 40 greater than of that from PBS.

Even more effective is LA/Ethanol, which is 50% ethanol (v/v) saturated with linoleic acid. Table 1 shows that the corticosterone permeability from LA/Ethanol is $8.7 \times 10^{-4}$ cm/hr. Note that all of the other formulations have lower permeabilities than from PBS, while the permeability from LA/Ethanol is nine-fold greater. The permeability enhancement achieved through the mere addition of linoleic acid to 50% Ethanol is 17-fold, clearly showing the effectiveness of the unsaturated fatty acid in increasing transport. Addition of linoleic acid to 50% Ethanol increases the corticosterone solubility to 12.4 mg/ml in LA/Ethanol from 9.2 mg/ml in 50% Ethanol alone, as shown in Table 1. Addition of the oily linoleic acid tends to make the solution more hydrophobic and slightly less polar, which is a more attractive environment for corticosterone. The combination of permeation enhancement and increased corticosterone solubility arising from the use of linoleic acid in 50% Ethanol combine to yield saturated drug fluxes of $1.1 \times 10^{-3}$ mg/cm$^2$/hr, which is 903-fold greater than from water and more than 20-fold greater than from 50% Ethanol (i.e., without the linoleic acid).

In order to examine the impact of linoleic acid on corticosterone transport without coupling it with 50% Ethanol, control experiments were performed in which corticosterone permeabilities were measured from PBS saturated with linoleic acid. The resulting corticosterone permeability, $1.1 \times 10^{-4}$ ($\pm 0.34 \times 10^{-4}$) cm/hr, is indistinguishable from the PBS permeability of $1.0 \times 10^{-4}$ ($\pm 0.32 \times 10^{-4}$) cm/hr reported in Table 1. Clearly, ethanol and linoleic acid are each ineffective in increasing corticosterone permeability when utilized individually, but when utilized together, they yield a substantial degree of enhancement.

## B. Effect of Ultrasound in Combination with Chemical Enhancers

Ultrasound was applied under therapeutically approved conditions (1.4 W/cm$^2$, 1 MHz, continuous) for 24 hours using a sonicator (Sonopuls 463, Henley International). The ultrasound transducer was located approximately 3 cm from the surface of the skin. Permeation experiments were performed using customized side-by-side diffusion cells having a skin area of 3.1 cm$^2$ and a receiver compartment volume of 7.5 ml. Samples were taken from the receiver compartment over 24 hours. The concentrations of radiolabelled drug in these samples, as well as in the donor compartment, were measured using a scintillation counter (model 2000 CA, Packard Instruments). Three or more experiments were performed using each of the chemical enhancers listed above. PBS was always used in the receiver compartment. Sonophoretic permeabilities were constant once steady-state was achieved. The drug permeabilities in the presence of ultrasound were $P_{us} = (dN_r/dt)/(AC_d)$. The exception to this observation was the combination of therapeutic ultrasound and SA/Ethanol, with which the corticosterone permeability continually increased.

Studies with therapeutic ultrasound 1 MHz performed at an intensity of 2.0 W/cm$^2$ by Mitragortri, et al. *J. Pharm. Sci.* 84, 697-706 (1995) showed that the continuous application of ultrasound increased transdermal permeabilities, but only for a short period of time. After 5 to 6 hours, the sonophoretic enhancement abated and the observed permeabilities returned to the passive values. This sonophoretic enhancement was found to be caused by cavitation within the skin, where cavitation is defined as the growth and oscillation of air bubbles which disorder the stratum corneum lipid bilayers. In the present study, ultrasound was applied at a lower intensity, 1.4 W/cm$^2$, and 1 MHz. Sonophoretic permeability enhancements lasted for extended periods of time for corticosterone, dexamethasone, and testosterone at this intensity. The elevated transdermal permeabilities resulting from the continuous application of ultrasound at 1.4 W/cm$^2$ were maintained for up to 48 hours, the longest sonophoretic experiment performed. As a control, the permeability of corticosterone was measured with therapeutic ultrasound applied at 2.0 W/cm$^2$. As was previously found and reported for estradiol, the permeation enhancement lasted for only 5 to 6 hours. This difference in the duration of the sonophoretic enhancements resulting from differences in the ultrasound intensity is probably due to the change in the magnitude of the cavitation activity. Since cavitation results in the degassing of the system, the greater ultrasound intensity results in an accelerated degassing of the system, which in turn results in shorter duration of the permeability enhancements, as was observed.

The transmission efficiency of ultrasound through the various enhancers was measured using a hydrophone (model PZT 54, Specialty Engineering Associates) coupled to a hydrophone preamplifier (model A17DB, Specialty Engineering Associates), and connected to an oscilloscope (model 7623 A, Hewlett Packard). The hydrophone was calibrated by Sonic Technologies. The ultrasound intensity in the diffusion cell was first measured with both probes submerged in the formulation and the hydrophone in close proximity to the ultrasound transducer. The ultrasound intensity was subsequently measured with the transducer in the donor chamber of the permeation cell and the hydrophone in the receiver chamber 5-6 cm away from the transducer. No differences in the measured intensities were observed for any formulation, indicating that all of the chemical enhancer formulations examined were uniformly efficient in transmitting ultrasound.

The uptake of $^{14}$C-linoleic acid into human SC was measured with and without the application of therapeutic ultrasound (1.4 W/cm$^2$, 1 MHz, continuous). SC was separated from heat stripped epidermis by soaking the epidermis in 0.5% Trypsin solution overnight at 5°C. The SC was cleaned with water, rinsed in cold hexane to remove any exogenous lipids, and lyophilized for at least 24 hours to remove all water. Dried pieces of SC were sectioned into pieces approximately 10 mg in weight and weighed. These SC pieces were place in a glass chamber mounted on an ultrasound probe containing 3 ml of solution of $^{14}$C-linoleic acid in LA/Ethanol and sealed. 25 μl samples were taken from the chamber periodically, and counted with the liquid scintillation counter.

Ultrasound is effective in increasing the permeability of corticosterone from all of the formulations examined, as shown in Table 2.

TABLE 2: Ultrasound-Mediated Permeability Enhancement of Corticosterone

| Enhancer | Permeability Without Ultrasound, $P$ (cm/hr x10$^5$) | Permeability With Ultrasound, $P_{us}$ (cm/hr x 10$^5$) | Ultrasound Enhancement $\varepsilon_{p,us}$ |
|---|---|---|---|
| Phosphate buffer | 10±32% | 50±39%* | 5.0 |
| PEG 200 Dilaurate | 2.4±29% | 4.5±24% | 1.9 |
| Isopropyl Myristate | 7.0±38% | 25±34% | 3.6 |
| Glycerol trioleate | 7.1±29% | 9.3±36% | 1.3 |
| 50% Ethanol 50% Buffer | 5.2±21% | 9.8±22% | 1.9 |
| Linoleic acid in 50:50 Ethanol:Buffer | 87±34% | ≥1260±50% | ≥14.4 |

*Experiments were performed on skin samples which had been utilized for passive permeability measurements, such that the lag-times were already surpassed.

The values of the sonophoretic permeability enhancements, $E_{p,\,us}$ defined as

$$\varepsilon_{\rho,us} \equiv \frac{P_{us}}{P} \tag{3}$$

are all greater than unity. Note that $E_{p,us}$, is the ratio of the ultrasound mediated permeability in a given formulation to the passive permeability in the *same* formulation, and hence is a measure of the effectiveness of ultrasound with that particular formulation. Table 2 shows that ultrasound mediated permeabilities for the first group of enhancers, PBS, PEG, IM, and GT, are all moderate, ranging from 1.3 for GT to 5.0 for water. The sonophoretic enhancement from 50% Ethanol, 1.9, is also moderate in its value. The most significant sonophoretic enhancement is obtained with the formulation containing linoleic acid, LA/Ethanol. The sonophoretic permeability from LA/Ethanol is $1.3 \times 10^{-2}$ cm/hr , which is a factor of 14 greater than the passive corticosterone permeability from LA/Ethanol. These results clearly show that ultrasound is effective in increasing transdermal drug permeation when utilized with both aqueous as well as non-aqueous formulations.

**Sonophoretic Saturated Fluxes and Enhancement**

The values of the ultrasound mediated corticosterone fluxes from saturated solution, $J_{us}$, where $J_{us} = P_{us}C^{sat}$, are listed in Table 3.

**TABLE 3: Enhancement of Corticosterone Transport by Chemical Enhancers and Ultrasound**

| Enhancer | Sonophoretic Saturated Flux, $J_{us}$ (mg/cm$^2$/hr x10$^5$) | Sonophoretic Saturated Flux Enhancement, $E_{J,us}$ |
|---|---|---|
| Phosphate buffer | 6.0 | 5.0 |
| PEG 200 Dilaurate | 4.2 | 3.5 |
| Isopropyl Myristate | 20 | 16 |
| Glycerol trioleate | 1.3 | 1.1 |
| 50% Ethanol 50% Buffer | 90 | 75 |
| 1 % Linoleic acid in 50:50 Ethanol:Buffer | ≥15,600 | ≥13,000 |

The fluxes from PBS, PEG, IM and GT are all fairly low, ranging from 1.3 x 10$^{-5}$ mg/cm$^2$/hr for GT to 6.0 x 10$^{-5}$ mg/cm$^2$/hr for PBS. The flux from PBS is greater than those from PEG, IM, and GT, due to greater sonophoretic permeability enhancement for PBS, shown in Table 2. While the flux from 50% Ethanol is 15-fold greater than that from PBS, 9.0 x 10$^{-4}$ mg/cm$^2$/hr, it is still a relatively low value. Table 3 shows that the use of LA/Ethanol and therapeutic ultrasound yields a flux greater than or equal to 0.16 mg/cm$^2$/hr, which is more than two orders of magnitude greater than that from 50% ethanol with ultrasound. Also listed in Table 3 are the sonophoretic saturated flux enhancements, $E_{J,us}$, which is defined as

$$(4) \qquad \varepsilon_{J,us} \equiv \frac{P_{us}(\text{enhancer})C^{sat}(\text{enhancer})}{P(\text{PBS})C^{sat}(\text{PBS})}$$

$E_{J,us}$ represents the flux enhancement relative to the passive flux from PBS, used to establish the base line. Moderate flux enhancements are observed for PBS, PEG, GT, and 50% Ethanol, ranging from 1.1 for GT to 75 for Ethanol. LA/Ethanol again is seen to provide tremendous flux enhancement, 13,000-fold more so than from passive PBS. This enormous enhancement is the result of the combination of ethanol, linoleic acid, and therapeutic ultrasound. Ethanol and water (1:1, v/v) greatly increases the saturated concentration of corticosterone (Table 1). Linoleic acid increases both the corticosterone solubility in 50% Ethanol as well as the corticosterone permeability, while ultrasound further increases the drug permeability when applied in conjunction with linoleic acid.

**C. Solubility measurements in chemical enhancers**

In separate studies, excess unlabeled corticosterone, dexamethasone, and testosterone were each placed in several milliliters of enhancer and thoroughly mixed. After equilibration for a minimum of 24 hours the solutions were removed, centrifuged at 1000 rpm (212 x g) for 10 minutes, and sampled. Samples were diluted to an appropriate concentration for high performance liquid chromatographic (HPLC) analysis utilizing the appropriate HPLC mobile phase. Methanol and water (60:40 v/v) was utilized as the mobile phase for corticosterone and testosterone, and acetonitrile and water (35:65 v/v) was utilized for dexamethasone. The mobile phases were filtered with 0.22 μm PTFE hydrophobic filters and degassed prior to usage. A μ-Bondapak C18 (30 cm x 4 mm, i.d.) HPLC column was used. The sample volume was 40 μl and the mobile phase flow rates were 1.4 ml/minute (corticosterone) and 2.0 ml/min. (corticosterone, dexamethasone, and testosterone). An ultraviolet detector (Waters 490) was used at a wavelength of 240 nm for all three drugs. Standards were prepared by diluting a stock solution of unlabeled drug, prepared by weight, with the mobile phases. Experiments performed in triplicate had a standard deviation of 1%.

TABLE 4: Passive and Ultrasound-Mediated Transport of Dexamethasone and Testosterone

| Drug | Enhancer | Passive Permeability $P$ (cm/hr x10$^5$) | Solubility $C^{sat}$ (mg/ml) | Saturated Flux, $J^{sat}$ (mg/cm$^2$/hr x 10$^5$) | Sonophoretic Permeability $P_{us}$ (cm/hr x10$^{5)}$ | Sonophoretic Saturated Flux, $J_{us}$ (mg/cm$^2$/hr x 10$^5$) |
|---|---|---|---|---|---|---|
| Dexamethasone | Phosphate buffer | 6.4±40% | 0.10 | 0.66 | | |
| | 50% Ethanol 50% Buffer | 1.7±23% | 2.39 | 4.0 | | |
| | Linoleic Acid in 50% Ethanol | 217±42% | 4.36 | 945 | 600±8% | 2610 |
| Testosterone | Phosphate buffer | 536±17% | 0.023 | 12.3 | | |
| | 50% Ethanol 50% Buffer | 5.5±8% | 6.37 | 35.2 | | |
| | Linoleic Acid in 50% Ethanol | 64±24% | 8.2 | 525 | 449±52% | 3680 |

EP 0 781 150 B1

In order to probe the generality of the effectiveness of LA/Ethanol alone and the combination of ultrasound and LA/Ethanol in enhancing corticosterone transport, experiments were performed with two additional model drugs, dexamethasone and testosterone. Passive permeability and solubility measurements were made with PBS, 50% Ethanol, and LA/Ethanol, as described above. Ultrasound mediated transport was also measured with LA/Ethanol and ultrasound with both dexamethasone and testosterone, as this was the most effective enhancement combination observed in the corticosterone experiments. The results of these experiments are shown in Table 4.

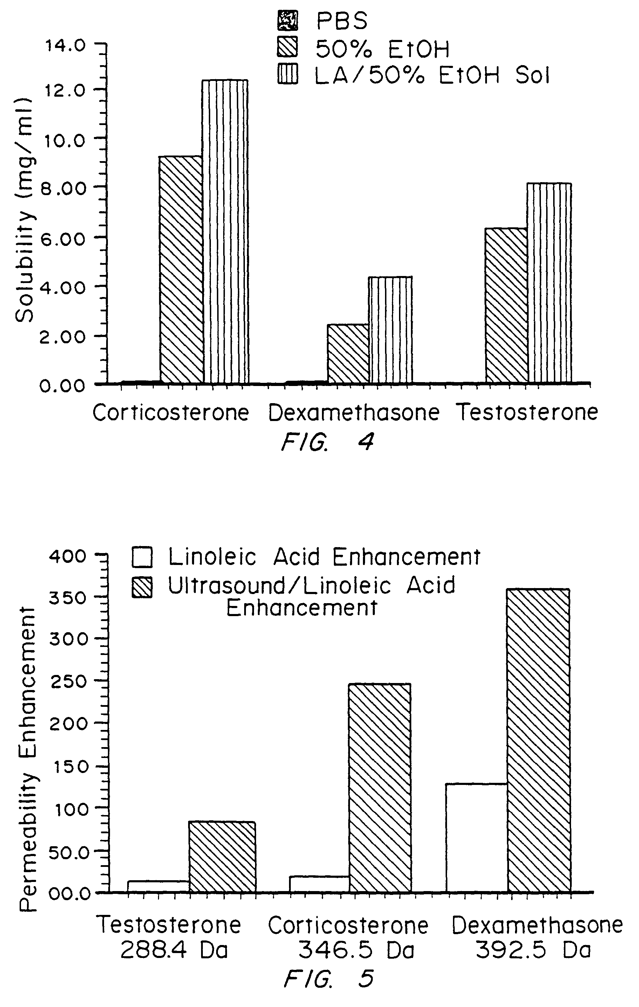

The solubility of dexamethasone in PBS is 0.10 mg/ml, which is similar to the solubility of corticosterone in PBS, 0.12 mg/ml. This is not surprising since dexamethesone and corticosterone have a similar degree of hydrophobicity, as revealed by their similar octanol/water partition coefficients of 97 for dexamethasone and 87 for corticosterone (Hansch and Leo, "Substitutent Constants for Correlation Analysis in Chemistry and Biology" (1979)). Testosterone is more hydrophobic than corticosterone and dexamethasone, as indicated by an octanol/water partition coefficient of 2100, has a lower solubility in PBS, 0.0234 mg/ml. Dexamethasone and testosterone are much more soluble in 50% Ethanol than PBS, 2.39 mg/ml and 6.37 mg/ml, and even more soluble in LA/ethanol, 4.36 mg/ml and 8.2 mg/ml respectively. The relative increases in drug solubility for all three drugs, corticosterone, dexamethasone, and testosterone, are shown in Figure 4. Solubilities of corticosterone, dexamethasone, and testosterone in PBS, 50% Ethanol, and LA/ethanol were measured using HPLC. The solubilities of these drugs in PBS, 0.12 mg/ml, 0.10 mg/ml, and 0.023 mg/ml respectively, are 77, 44, and 360 fold lower than the solubilities in 50% Ethanol. Corticosterone, dexamethasone, and testosterone are even more soluble in 50% Ethanol saturated with linoleic acid (LA/Ethanol) by an average factor of 1.5.

The experimentally measured permeability of dexamethasone from PBS is $6.4 \times 10^{-5}$ cm/hr, as shown in Table 4. This value is relatively low, yet the permeability from 50% Ethanol is even lower at $1.7 \times 10^{-5}$ cm/hr. The permeability of testosterone from PBS is $5.4 \times 10^{-3}$ cm/hr, but decreases by nearly two orders of magnitude to a value of $5.5 \times 10^{-5}$ cm/hr when measured from 50% Ethanol. Similar drops have been observed for corticosterone, as shown in Table 1, as well as for estradiol (Liu, et al. *Pharmaceutical Research 8,* 938-944 (1991)). These permeability decreases are a result of the decreased partitioning of the drugs into the skin. Since 50% ethanol has a lesser degree of polarity than does water, it is more attractive environment relative to PBS, and shifts the equilibrium drug distributions away from the skin and towards the donor solution. Since the skin permeability is proportional to the partition coefficient, skin permeabilities will decrease as the donor solution becomes less polar than PBS and a better solubilizer of the drugs. The permeability of dexamethasone from LA/Ethanol, $2.2 \times 10^{-3}$ cm/hr, is significantly greater than that from PBS, $6.4 \times 10^{-5}$ cm/hr, as was also observed with corticosterone. However, the permeability of testosterone from LA/Ethanol, $6.4 \times 10^{-3}$ and $5.3 \times 10^{-3}$ mg/cm$^2$/hr, are within a factor of two of the corticosterone saturated flux, $1.1 \times 10^{-2}$ mg/cm$^2$/hr.

Dexamethasone and testosterone sonophoretic transport measurements were made with LA/Ethanol, which is the most effective combination that was determined through the corticosterone experiments. The results of these experiments are shown in Table 4. As with corticosterone, the sonophoretically enhanced permeabilities of dexamethasone and testosterone increased over time. However, unlike the corticosterone experiments, these observed permeabilities become steady after 12 to 15 hours, enabling true steady-state measurements to be made: $6.0 \times 10^{-3}$ cm/hr and $4.5 \times 10^{-3}$ cm/hr for dexamethasone and testosterone, respectively. As with corticosterone, tremendous sonophoretic saturated fluxes of $2.6 \times 10^{-2}$ mg/cm$^2$/hr and $3.7 \times 10^{-2}$/hr are obtained for dexamethasone and testosterone.

Figure 5 shows that the permeation enhancement resulting from the use of linoleic acid is dependent upon the drug examined and the size of that drug. Permeability enhancements for testosterone (288.4 Da), corticosterone (346.5 Da), and dexamethasone (392.5 Da) through (1) the addition of linoleic acid to 50% ethanol and (2) the addition of linoleic acid to 50% ethanol with the continuous application of therapeutic ultrasound relative to the permeabilities from 50% ethanol alone were observed. The enhancements from linoleic acid bear a distinct size dependence, with the larger compounds having larger enhancements. Enhancements with linoleic acid and therapeutic ultrasound bears an analogous size dependence with greater enhancements observed for larger compounds. The permeation enhancements resulting from linoleic acid, (the ratio of the permeability from LA/Ethanol and the permeability from 50% ethanol) alone, are 12 for the smallest drug (testosterone, 288.4 Da), 17 for corticosterone (346.5 Da), and 130 for the largest drug (dexamethasone, 392.5 Da).

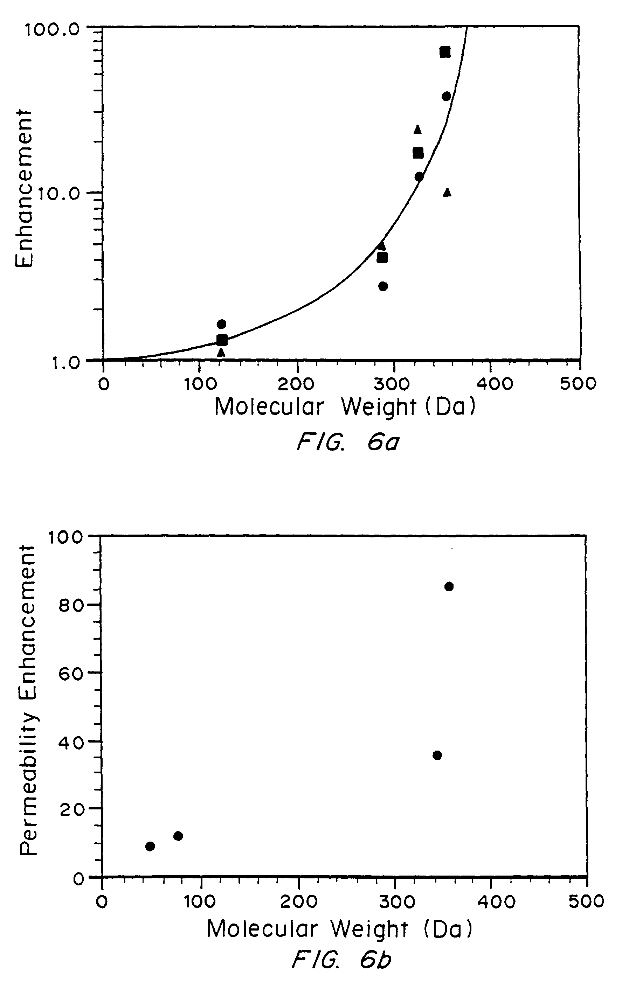

The enhancement effects of three different enhancers, capric acid (J), lauric acid (B), and Neodecanoic acid (H), upon the human skin permeabilities of benzoic acid (122 Da), testosterone (288 Da), naloxone (328 Da), and indomethacin (359 Da), as compared with propylene glycol are shown in Figure 6a. These enhancements exhibit a clear size dependence, with the larger compounds being enhanced to a greater degree than the smaller compounds. The line is drawn to guide the eye. The permeability values were originally reported by Aungst et al. Figure 6a shows the permeability enhancements plotted as a function of the molecular weight of the drug. Figure 6a also shows that the variations in enhancement of a given drug from using the different enhancers extends to be less than the variation of enhancements between the different drugs.

The enhancement of Azone upon the permeabilities of ethanol (46 Da), butanol (74 Da), corticosterone (346 Da), and hydrocortisone (362 Da) from aqueous solutions through hairless mouse stratum corneum are shown in Figure 6b. The skin was pretreated by spraying 0.8 mg/cm$^2$ of Azone upon it. The degree of permeability enhancement correlates with the size of the solute. The permeability values were originally reported in graphical form by Lambert et al.

Fluidization of the stratum corneum lipid bilayers can increase the partition coefficient between the bilayers and the donor medium in addition to increasing diffusion. Since partitioning is a function of the chemical nature of a solute (i.e., the hydrophobic/hydrophilic nature of a solute) and not an independent function of molecular weight, the partitioning effect would only tend to obscure the size dependence of enhancement. Linoleic acid and the other chemical enhancers may increase drug transport through alternate pathways, often referred to as aqueous pores. The passive skin permeabilities of hydrophilic compounds, which are thought to diffuse through these aqueous pores, exhibit a much weaker size dependence than that of hydrophobic compounds. While this size dependence is moderate, as are those for fluid phase phospholipid bilayers and bulk oil phases, fatty acids have been shown to interact predominantly with the intercellular lipids. Ethanol alone can also enhance transport of both hydrophobic and hydrophilic compounds through these aqueous pore pathways, although high concentrations of ethanol are needed (i.e. approximately 75% v/v). Ghanem et al., *Int. J. Pharm.* 78, 137-156 (1992) also report that lipoidal compounds in solutions of 50% ethanol or less permeate the SC primarily through the lipoidal domain. This indicates that the combination of 50% ethanol and linoleic acid may make the aqueous pore pathway more effective. If the linoleic acid worked with the 50% ethanol solution to facilitate aqueous pore transport, the passive permeabilities from LA/Ethanol would be expected to be essentially constant and independent of size. Tables 1 and 4 show that this is not the case.

The results of sonophoretic enhancement experiments conducted over the last four decades for more than a dozen different drugs, ranging in size from 138 Da (salicylic acid) up to 453 Da (fluocinolone acetonide) were collated. These studies include both in vitro and *in vivo* experiments. While some studies quantified the degree of enhancement, others simply reported whether or not sonophoretic enhancement was observed. No sonophoretic enhancement has been observed for drugs smaller than 250 Da whereas sonophoretic enhancement has been observed for compounds larger than 250 Da, with the lone exception of progesterone. Sonophoretic enhancement correlates very well with the drug passive diffusion coefficient, which in turn is a strong function of molecular weight.

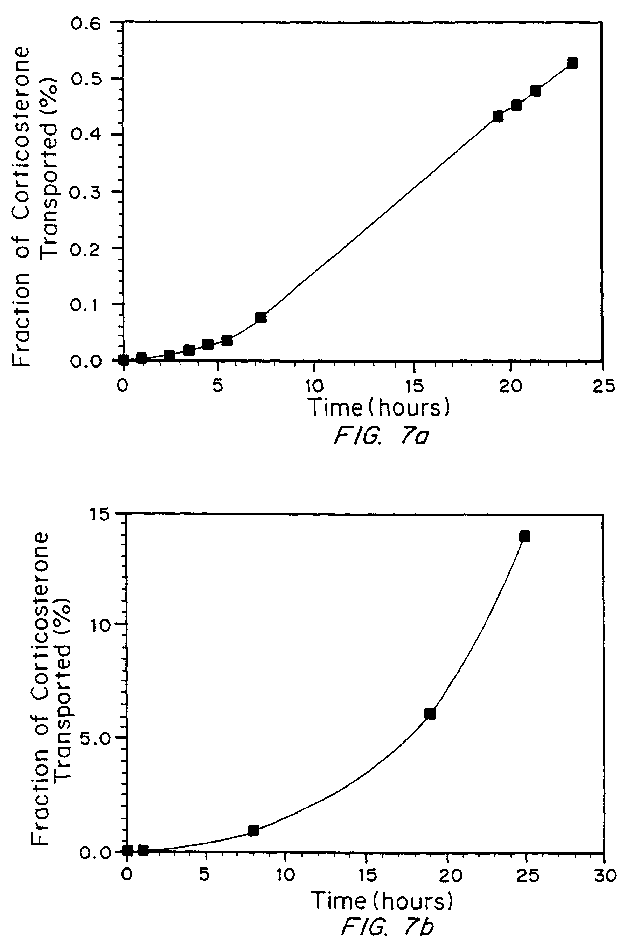

Figures 7a and 7b graph the variation of the permeability over time of the corticosterone permeability through human skin from (Figure 7a) PBS and (Figure 7b) LA/Ethanol in the presence of therapeutic ultrasound (1 MHz, 1.4 W/cm$^2$). The corticosterone permeability from PBS is maintained at the steady-state value of the duration of the 24 experiment once the lag-time period is surpassed. The corticosterone permeability from LA/Ethanol, on the other hand, continues to increase over time. The typical error of the data points is 3%. The lines are drawn to guide the eye.

The sonophoretically enhanced permeabilities were constant over time with PBS, PEG, IM, GT, and 50% Ethanol, as shown in Figure 7a for a typical corticosterone experiment from PBS. After an initial lag time of several hours, the permeability remains constant for the duration of the 24 hour experiment. However, when ultrasound was applied in conjunction with LA/Ethanol, the corticosterone permeability continually increased. Figure 7b shows the results of one such experiment, wherein the fraction of corticosterone transported across the skin is plotted versus time. Whereas steady-state conditions are defined by linear slope on such a plot, the slope in Figure 7b continually increases. This continual increase in corticosterone permeability was observed in every ultrasound mediated experiment performed with corticosterone and the LA/Ethanol formulation (n=6).

A steady-state permeability can not be directly measured in the ultrasound mediated permeation experiments with LA/Ethanol due to the lack of a linear profile in Figure 7b. The permeability value listed in Table 2 for this condition is the average of the permeabilities observed at the 24 hour mark of the experiment. While this value is not a steady-state permeability, it does constitute a *lower bound* on the true steady-state permeability. This value, $1.3 \times 10^{-2}$ cm/hr, is large relative to the other corticosterone permeabilities, and is 126-fold greater than the passive permeability from PBS alone. The true steady-state permeability is greater than or equal to $1.3 \times 10^{-2}$ cm/hr and the sonophoretic enhancement is greater than or equal to 14. The slightly elevated error associated with this value, a standard deviation of 50%, is also a result of the fact that the permeabilities were calculated from non-linear portions of the flux profiles.

The ultrasound mediated experiments performed with 50% ethanol (without linoleic acid) exhibited a constant permeability after the initial lag time. The results shown in Figure 7b are dependent upon the combined application of linoleic acid and therapeutic ultrasound. This relationship was further probed in a set of experiments with corticosterone in LA/ethanol in which ultrasound was applied for the first eight hours of the experiments, after which the ultrasound was turned-off. Variation of the permeability over time of the corticosterone permeability through human skin from LA/Ethanol with the discontinuous application of therapeutic ultrasound (1 MHz, 1.4 W/cm$^2$) was measured. Ultrasound was applied for the first eight hours of the experiment, at which time it was turned off. In contrast with the results of Figure 7b in which ultrasound was applied continuously and the permeability continued to rise for the entire 24 hour period, the corticosterone permeability increases up to the point at which the ultrasound is discontinued, at which point it remains constant for the remainder of the experiment. The typical error of the data points is 3%.

**EXAMPLE 5:**     **Transdermal drug delivery and extraction of gluose using ultrasound in combination with additional force fields.**

**Materials and Methods:**

*In vitro* experiments were performed to study the effect of ultrasound in combination with electric currents on the delivery of calcein and extraction of glucose across human cadaver skin. The skin was heat stripped by keeping it in water at 60°C for two minutes, followed by the removal of the epidermis. The skin was then stored at 4°C in a humidified chamber. A piece of epidermis was taken out from the chamber prior to the experiments, and was mounted on a Franz diffusion cell (Crown Bioscientific Co.) which consists of a donor and a receiver compartment. The skin was supported by a nylon mesh (Tetko Inc.) to minimize mechanical oscillations during ultrasound application. The donor and receiver compartments were then clamped together. Two Ag/AgCl electrodes were introduced in the donor and the receiver compartment for the application of electric fields. The receiver compartment was filled with a solution of radiolabeled glucose in phosphate buffer saline (Sigma Chemicals), and the donor solution was filled with a calcein solution (Sigma Chemicals).

The ultrasound transducer was located at a distance of about 1 cm from the skin. The ultrasound was turned ON at a frequency of 20 KHz and intensity of 125 mW/cm$^2$ for 10 minutes. Electric current (0.1 mA/cm$^2$) was applied across the skin in a few experiments for 70 minutes with anode (electrode carrying the positive charge) inserted in the receiver compartment and the cathode (electrode carrying the negative charge) in the donor compartment. The concentration of calcein in the donor and the receiver compartment was measured using the spectrofluorimeter (Photon Technology Int). The concentration of glucose in the donor and the receiver compartment was measured using scintillation counter.

**Results:**

**a) Transdermal Calcein Transport:**

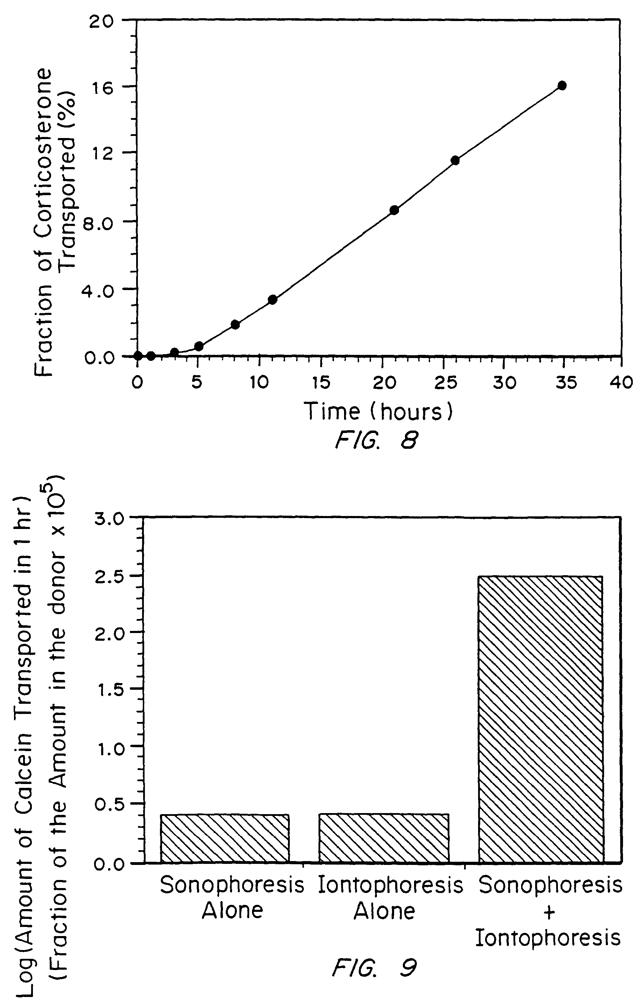

Calcein possesses a molecular weight of 622 and a net charge of -4. Due to its charge and relatively large size, passive transdermal transport of calcein is extremely low. Figure 9 shows the transdermal transport of calcein during sonophoresis, iontophoresis or combination thereof. Sonophoresis alone at 20 KHz and 125 mW/cm$^2$ for 10 minutes followed by a waiting period of 1 hour (total time of 70 minutes) resulted in transdermal transport of about 3.2 x 10$^{-3}$ % of calcein present in the donor compartment. Similarly, application of electric current (0.2 mA/cm$^2$) alone for 70 minutes induced transdermal transport 2.5 x 10$^{-3}$ % of calcein present in the donor compartment. A combination of the two methods: 10 minutes of sonophoresis (20 KHz, 125 mW/cm$^2$) and 70 minutes of *simultaneous* iontophoresis (0.2 mA/cm$^2$) (ultrasound and electric current ON for the first 10 minutes with only iontophoresis ON for the next 60 minutes) resulted in transdermal transport of about 3.5 x 10$^{-1}$% of calcein present in the donor compartment. The results show that transdermal calcein transport during a combined treatment of sonophoresis and iontophoresis is about 100-fold higher than that during sonophoresis or iontophoresis under similar conditions.

**b) Transdermal Glucose Extraction:**

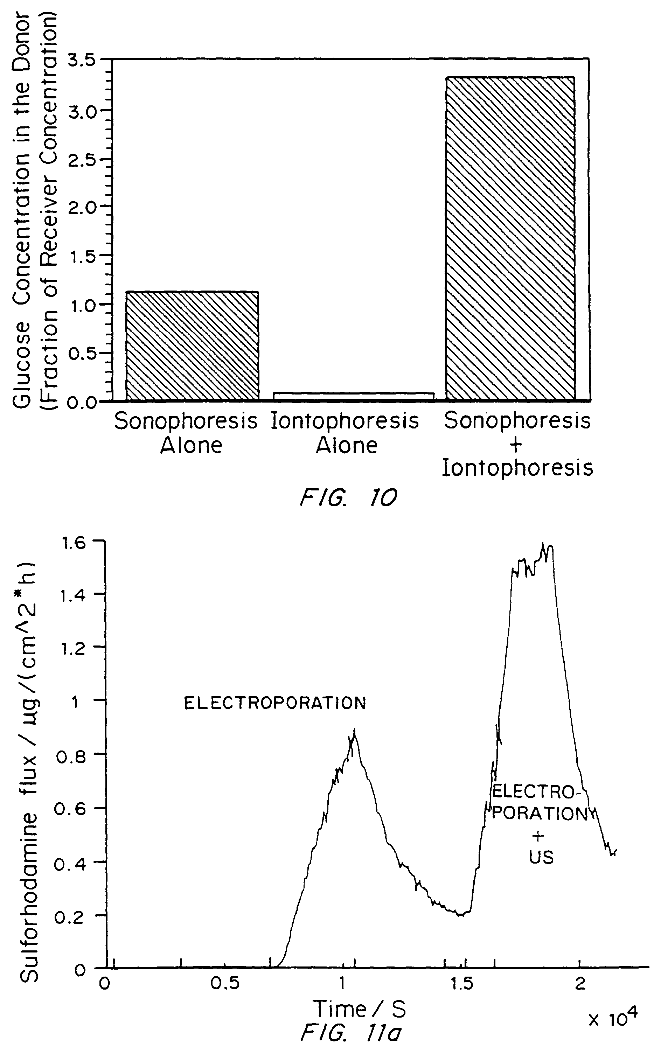

Glucose is a hydrophilic molecule and shows no detectable transdermal transport passive diffusion. Figure 10 shows the amount of glucose extracted transdermally by sonophoresis, iontophoresis or combination thereof. Application of ultrasound (20 KHz, 125 mW/cm$^2$) alone for 10 minutes followed by a waiting period of 1 hour (total time of 70 minutes) resulted in the donor glucose concentration of about 1% of the receiver glucose concentration. Application of electric current (0.2 mA/cm$^2$) (ultrasound and electric current ON for the first 10 minutes with only iontophoresis ON for the next 60 minutes) resulted in a donor glucose concentration which was about 3.4% of the receiver concentration. Simultaneous application of sonophoresis and iontophoresis induced about threefold higher transdermal glucose transport than that induced by sonophoresis alone.

**EXAMPLE 6:**     **Comparison of drug transfer through skin using ultrasound or electrical field alone or in combination.**

**MATERIALS AND METHODS**

**A.     Materials**

Full thickness of human cadaver skin (obtained from local hospitals) was heat stripped by immersion in 60°C water for two minutes followed by the removal of the epidermis. The skin was then stored in a humidified chamber (95% relative humidity) at 4°C. The heat-stripped human epidermis was placed in a custom-made side-by-side permeation chamber, skin area of 0.64 cm$^2$, designed to adapt an ultrasound transducer at the donor side. The donor compartment was filled with a 1 mM solution of calcein (MW 623, electric charge - 4; Sigma Chemicals) (CA) and 1 mM sulphorhodamine (MW 607, electric charge - 1; Sigma Chemical) (SR) in 150 mM Phosphate Buffer Saline (PBS; Sigma Chemicals).

The ultrasound probe was inserted into the donor compartment. The direction of the ultrasound wave was perpendicular to the membrane surface. The stratum corneum was facing the donor compartment. Both donor and receptor compartments were filled with degassed phosphate buffer saline (PBS) pH=7.4. The temperature was followed to be in the range of 22 $\pm$ 2°C. SR and CA were added to the donor compartment to provide concentration of 1 mM CA and 1 mM SR. Fresh PBS was continuously pumped into the receptor compartment at 0.8 ml/min from a reservoir.

## B.    Fluorescence measurements

The fluorometer was set up for dual wavelength measurements (excitation wavelength = 488 nm, emission wavelength = 515 nm (calcein), and excitation wavelength = 586 nm, emission wavelength = 607 nm (sulphorhodamine)). The sample cuvette of the fluorometer was sealed but for two openings that were provided for the flow of receiver fluid through it. A small custom-made electric stirrer was installed in the cuvette so that there were no stagnant zones in it. Care was taken to avoid any obstruction of the excitation beam by the stirrer. Transdermal calcein and sulphorhodamine flux was calculated from the fluorescence readings by taking into account parameters such as flow rate, receiver compartment volume, and fluorometer caveat volume. The effluent from the receptor compartment was pumped through a spectrofluorometer (Fluorolog-II-system F112AI SPEX-industries, Edison, NJ) where the fluorescence of calcein and sulphorhodamine was separately measured twice every minute. The excitation for CA is 488 nm and for SR 586 nm, the measurement for CA was at 515 nm and for SR 607 nm. The receptor was mixed by an electromechanical stirrer. The fluorescence measurements were deconvoluted to calculate the CA and SR flux.

## C.    Application of Ultrasound

Two studies were conducted. In the first, two ultrasound sources were utilized:

i. 20 KHz Sonics and Materials (250 W) with a probe surface area of 0.25 cm$^2$.
ii. 1 MHz Sonopuls Therapeutic device with probe surface area of 0.8 cm$^2$. Pulsed and continuous modes were evaluated below 2 W/cm$^2$ for the continuous mode and 2-3 W/cm$^2$ pulsed (20% duty-cycle). The distance of the probe tips from the skin was 3 cm for the 20 KHz and 4 cm for the 1 MHz.

In the second study, ultrasound was applied under therapeutically approved conditions (1.4 W/cm$^2$, 1 MHz and 3 MHz, continuous) using a sonicator (Sonopuls 463, Henley International) for various exposure times up to 1 hour. The ultrasound transducer was located at a distance of about 3 cm from the skin.

## D.    Electroporation

One Ag/AgCl electrode (In vivo metric, Healdsburg, CA) was located in the donor and one in the receptor compartment, so that the distance of electrodes from the skin was equal in both the compartments (about 8 mm). Voltage pulses were applied using a pulse generator (ECM 600, BTX, San Diego, CA) across the electrodes such that the positive electrode was always in the receptor compartment. This provided an electric driving force for calcein and sulphorhodamine (both negatively charged) to transport across the skin. The voltage applied to the electrodes divides between the saline and the skin. The voltage drop across the skin was estimated using the measured electrical resistance of the skin and saline. The magnitude as well as the length of the voltage pulses was varied over a wide range in order to investigate their effect on transdermal transport.

In the first set of experiments (Figures 11a 11b, 11c a voltage divider of 10:40 ohm was used to provide a fixed time constant (exponential shape pulse). The maximum pulsing voltage in all experiments was 750 volts across the chamber (refers to a voltage drop across the skin of 210 - 230 volts). The pulse rate was 1 pulse/min for 60 minutes, controlled by a computer.

In the second set of studies (Figures 11c, 12b and 12c, 13), the electric field (100 V) was applied across the skin, exponentially decaying pulse with a time constant ($\tau$) of 1 millisecond, one pulse applied every minute.

In order to assess the stability of these molecules during electroporation, calcein and sulphorhodamine solutions (1 mM each) were exposed to electroporating conditions similar to those used in this study. No difference between the intensity of their fluorescence before and after exposure to electric fields could be detected. In addition, these molecules are stable up to a temperature of 100°C (measured in terms of fluorescence). When these molecules are degraded, they do not fluoresce. In general, these molecules have been found to be very stable against many physicochemical changes.

**E.    Measurements of Passive Electric Skin Properties**

A second pair of electrodes (same type as above) was used for monitoring the passive electrical properties (specifically, electrical resistance). Since the electrical resistance of the skin is a good indicator of its barrier properties, the skin resistance was measured before, during and after the experiments. The effect of electroporation and ultrasound separately and together on skin electrical resistance was determined. If the electrical resistivity before the application of either ultrasound or electroporation was lower than 20 kΩ-cm$^2$ or if any significant passive calcein or sulphorhodamine transdermal flux was observed (that is, J greater than 0.002 μg/cm$^2$/h (the detection limit)), the skin piece was considered leaky and replaced by a new piece.

**RESULTS AND DISCUSSION**

**A.    Application of Ultrasound Enhances the Efficacy of Electric Field.**

The results of the first study are shown in Figure 11a. Figure 11a shows the time variation flux of SR which permeated the skin with time. After 400 sec of passive diffusion, pulsed ultrasound (1 MHz, 20% duty cycle, 2.5 - 2.9 W/cm$^2$) was turned on for 2750 sec. The ultrasound was turned off at 2750 sec. High voltage pulsing was turned on at 6900 sec for 1 hour (10,500 sec end of electroporation pulsing). Ultrasound (1 MHz, 0.8 cm$^2$ 20% duty cycle, 2.5 - 2.9 W/cm$^2$) was turned on again at 14,310 sec, electroporation (same condition) was turned on again at 15,200 sec while the pulsed ultrasound was on. At 16,440 sec the ultrasound wave was changed from pulsed to continuous while the electroporation continued.
The experiment was terminated at 20,040 sec. The experimental procedure is summarized in Table 5 below.

TABLE 5:

| Conditions used for determining effect of ultrasound and electroporation | | |
|---|---|---|
| From (sec) | To (sec) | Description of the transdermal transport |
| 0 | 400 | passive diffusion |
| 400 | 3150 | pulsed ultrasound |
| 3150 | 6900 | passive diffusion |
| 6900 | 10500 | electroporation |
| 10500 | 14310 | passive diffusion |
| 14310- | 15200 | pulsed ultrasound |
| 15200- | 16400 | electroporation + pulsed ultrasound |
| 16440- | 20040 | electroporation + continuous ultrasound |

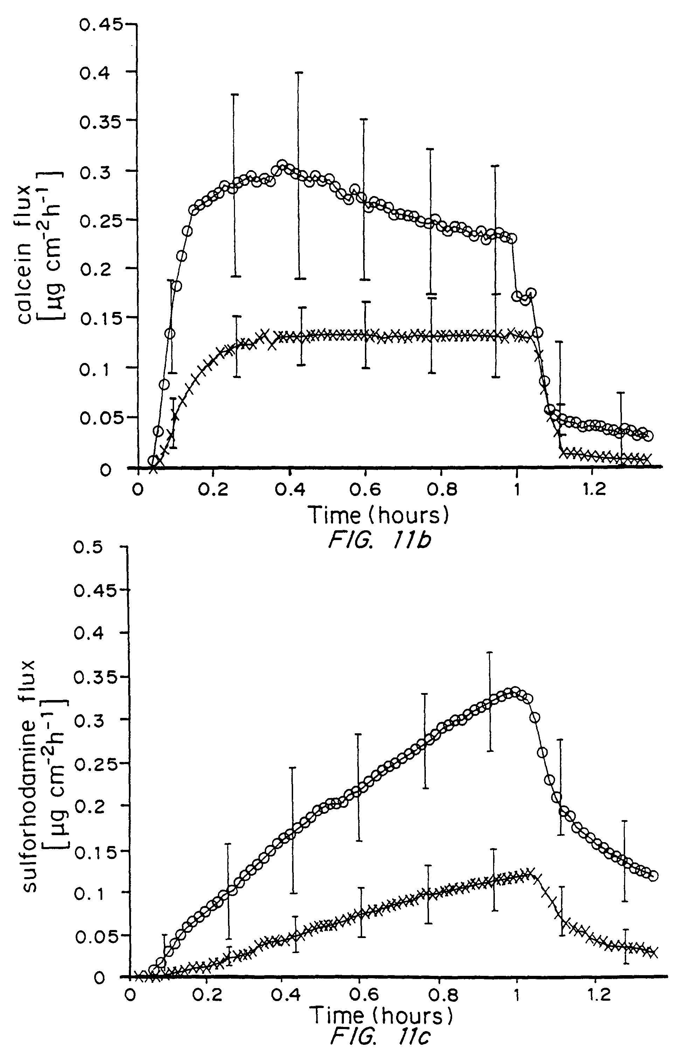

Figure 11b and 11c show the effect of simultaneous application of ultrasound (1 MHz, 1.4 W/cm$^2$, continuous application) and electric field (100 V across the skin, exponentially decaying pulse with a time constant (τ) of 1 millisecond, one pulse applied every minute) on the transdermal transport of calcein and sulphorhodamine respectively. The passive transdermal transport (in the absence of ultrasound and electric field) is below the detection limit and is not shown in Figure 11b or 11c. Application of ultrasound alone does not enhance the flux of calcein or sulphorhodamine. However, application of ultrasound enhanced steady-state transdermal flux of both calcein and sulphorhodamine during electric field pulsing. The enhancement is quantitatively defined as the amount of calcein or sulphorhodamine transported in the presence of ultrasound-electric field pulsing to that in the presence of electric field pulsing alone. This ratio is 2 in the case of calcein (Figure 11b) and 3 in the case of sulphorhodamine (Figure 11c Application of ultrasound also reduced transdermal calcein transport lag time, defined as the time required to reach the steady state, from a typical value of 15 minutes in the presence of electric field alone to about 9 minutes in the presence of ultrasound and electric field.

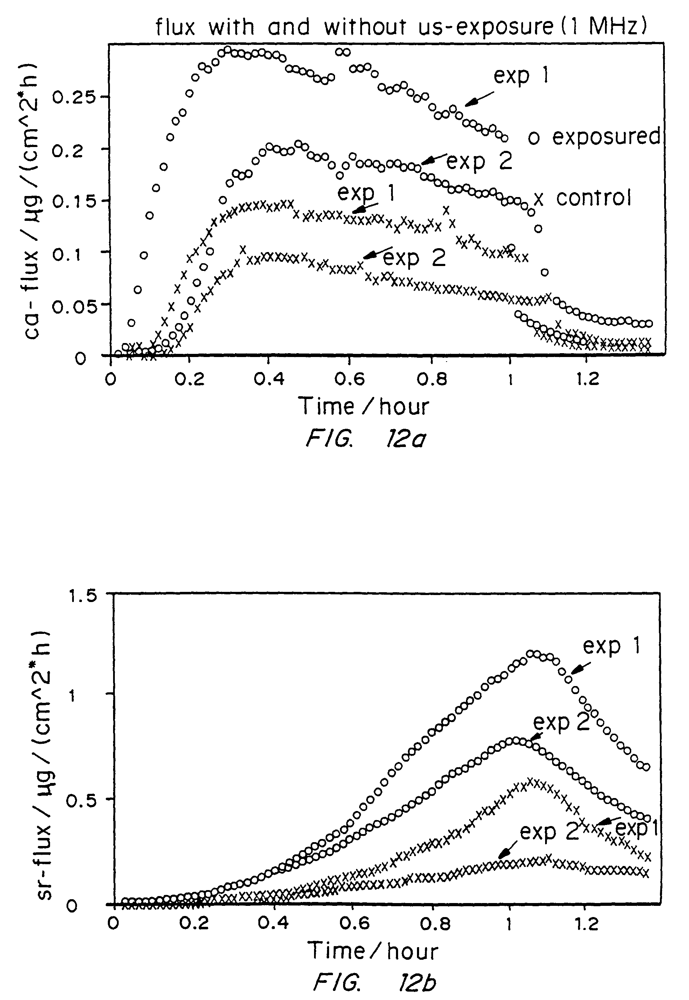

Similar effects of ultrasound on transdermal transport of SR and CA during electroporation can be also seen in Figures 12a and 12b which present the flux of CA (Figure 12a) and SR (Figure 12b) in experiments where the skin samples were exposed continuously to electroporation and continuous ultrasound (1 MHz, 0.8 $cm^2$, 2 $W/cm^2$) (o) and controls (x) where the skin was exposed to electric fields alone. The possible mechanism for this phenomena might be that the electrical pulsing creates short term pores in the skin while ultrasound is forcing the solutes through these pores.

In order to quantitatively estimate the reduction in the required pulsing voltages by simultaneous application of ultrasound and electric field, transdermal sulphorhodamine transport was measured in the presence as well as absence of ultrasound (1 MHz, 1.4 $W/cm^2$) and electric field (voltage across the skin increased from 20 V to 150 V in steps of 5 V every 30 minutes, 1 millisecond exponential pulse applied every minute).

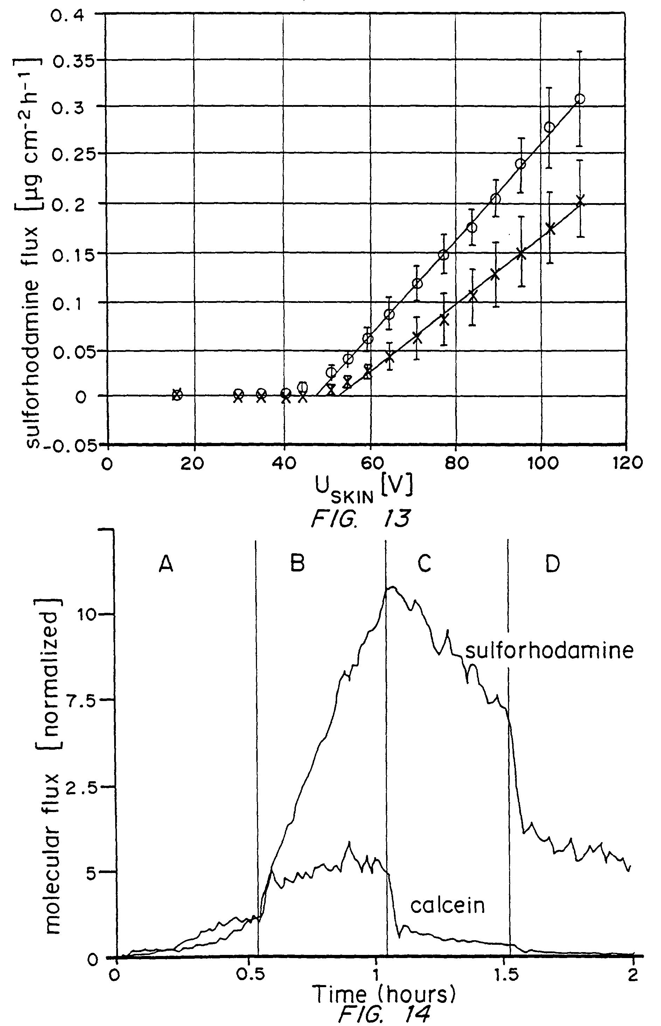

Figure 13 shows the variation of transdermal sulphorhodamine flux with voltage across the skin in the presence (O) as well as in the absence (X) of ultrasound. The transdermal sulphorhodamine flux is nearly zero as long as the voltage is below the threshold value and thereafter increases linearly with voltage. The threshold voltage for this pulsing protocol can be estimated by measuring the intercept of the linear variation of flux with voltage on the voltage axis. In the absence of ultrasound, this threshold is about 53 $\pm$ 3 V and that in the presence of ultrasound is about 46 $\pm$ 3 V, indicating that application of ultrasound slightly reduces the threshold pulsing voltage. Figure 13 also shows that the transdermal sulphorhodamine flux at various pulsing voltages is always higher in the presence of ultrasound. Thus, the pulsing voltage required to achieve a given transdermal flux is smaller in the presence of ultrasound. For example, to achieve a transdermal sulphorhodamine flux of 0.15 $\mu g/cm^2/hr$, the required voltage is about 95 V in the absence of ultrasound and 75 V in the presence of ultrasound.

Cavitation may play a two-fold role in enhancing the effect of electric field on transdermal transport. Since the electrical resistance of the disordered bilayers is likely to be smaller than that of the normal lipid bilayers, the applied electric field may concentrate preferentially across the normal bilayers. This may decrease the threshold electroporating voltage for transdermal transport of calcein and sulphorhodamine. Application of ultrasound reduces the threshold pulsing voltage from about 53 $\pm$ 3 V in the absence of ultrasound to about 46 $\pm$ 3 V in the presence of ultrasound (a reduction of about 12%). This number is comparable to an independent estimate of the fraction of SC bilayer disordered by ultrasound application (15%).

The oscillations of cavitation bubbles may also induce convection across the skin. In order to assess the role of convection in the synergistic effect of ultrasound and electric field, transdermal calcein and sulphorhodamine transport was measured sequentially in the presence of electric field alone, ultrasound and electric field, ultrasound alone and in the absence of ultrasound and electric field. The results of these sequential procedure are shown in Figure 14. Results from a single experiment are shown to depict the shape of the curves clearly. Note the change in the transdermal flux at 1 and 1.5 hours when electric field and ultrasound is turned OFF respectively. If electrophoresis plays an important role in calcein and sulphorhodamine transport, the transdermal flux is likely to decrease rapidly after *electric fields* is turned OFF. On the other hand, if cavitation-induced convection plays an important role, transdermal flux would rapidly decrease after turning *ultrasound* OFF. Indeed, calcein flux decreases rapidly after turning electric field OFF (1 hour) and achieves a value comparable to the background flux. When ultrasound is turned OFF at 1.5 hours, calcein flux further decreases by a small amount (compared to the reduction after turning electric field OFF at 1 hour) and thereafter it remains nearly at the background level. This suggests that calcein transport is mainly driven by electric forces. On the other hand, convection appears to play an important role in transdermal sulphorhodamine transport in the presence of ultrasound and electric field because the sulphorhodamine flux *did not decrease rapidly* after turning electric fields OFF, but decreased *instantaneously* after turning ultrasound OFF at 1.5 hours. The total decrease in the transdermal sulphorhodamine flux after turning the electric field OFF (that is, between a period of 1 and 1.5 hours) is comparable to the instantaneous decrease in its value after turning ultrasound OFF at 1.5 hours. This suggests that both electric field and ultrasound-generated convection may play an important role in transdermal sulphorhodamine transport. This difference in the behavior of calcein and sulphorhodamine is presumably because calcein possesses a much larger charge (-4) compared to sulphorhodamine (-1). In this respect, it is important to note that the transdermal transport of calcein and sulphorhodamine in the presence of electric field alone also differs significantly. Calcein transport increases rapidly and achieves a steady state within 15 minutes. Sulphorhodamine flux, however, increases continuously with time over the experimental duration. This difference in the behavior of calcein and sulphorhodamine flux may also be attributed to the lower charge on sulphorhodamine, as the transport during the electrical pulses is driven by electrophoresis.

The combined effect of electroporation and ultrasound on transdermal flux in all experiments was higher for SR than CA, suggesting that the additional enhancement by ultrasound is more effective on less charged molecules. The effect of ultrasound was observed on both the lag time and the steady state flux for the two molecules.

In summary, electroporation of the skin resulted in a very significant increase in SR permeability. The phenomenon was observed also on repeated application of electroporation, but the enhancing effect was only slightly higher. Application of ultrasound without electroporation did not result in enhanced flux. The very pronounced increase in permeability was observed when the skin was exposed to the combined effect of ultrasound and electroporation (more than twice the flux value observed with electroporation without ultrasound). The combined effect of ultrasound and electroporation was also observed in additional exposures of the same skin specimens.

**EXAMPLE 7:**     **Determination of effect of ultrasound on skin.**

The following experiment was measured in order to assess whether application of ultrasound induces any irreversible change in the skin structure. Human skin pieces were exposed to electric field alone (100 V across the skin, exponentially decaying pulse with a time constant ($\tau$) of 1 millisecond, one pulse applied every minute), then simultaneously to ultrasound (1 MHz, 1.4 W/cm$^2$)-electric field and again to electric field alone. A comparison of sulphorhodamine transport due to the electric field alone, before and after the simultaneous electric field-ultrasound treatment, indicated that the flux returns to a near baseline value, suggesting that the application of ultrasound did not induce any irreversible alteration in the barrier properties of skin. The recovery was also supported by electric resistance measurements indicating that application of ultrasound did not cause any irreversible change in the electrical resistance of the skin.

**Claims**

1.  A method for extracting an analyte to be measured in a blood or body fluid sample comprising applying at an appropriate site for extraction of a sample an effective amount of ultrasound and collecting the extracted sample, **characterised in that** the ultrasound for transporting the molecules of the sample is applied at a frequency between 20 kHz and less than 1 MHz.

2.  An apparatus for enhancing transdermal transport of molecules comprising means for administering to the skin an effective amount of ultrasound for transdermal transport of molecules **characterised in that** the ultrasound for transporting the molecules has a frequency of less than 2.5 MHz and the apparatus further comprises an enhancer selected from the group consisting of chemical enhancer combinations of agents enhancing solubility of the molecules to be transported with agents enhancing the fluidity of lipid bilayers, mechanical force fields, osmotic force fields, magnetic force fields and electric force fields.

3.  The apparatus of Claim 2 which comprises means for administering ultrasound at a frequency of 1 MHz or less.

4.  The apparatus of Claim 2 or Claim 3 wherein the enhancer is linoleic acid in an ethanol solution.

5.  The apparatus of Claim 2 which comprises means for administering ultrasound in combination with an electric force field selected from the group consisting of electroporation and iontophoresis.

6.  The apparatus of Claim 5 wherein the electric field is pulsed.

7.  The apparatus of Claim 2 which comprises means for administering ultrasound in combination with a magnetic force field.

8.  The apparatus of Claim 2 which comprises means for administering ultrasound in combination with a mechanical force field created by pressure or vacuum.

9.  The apparatus of Claim 2 which comprises means for administering ultrasound in combination with an osmotic force field.

10. The apparatus of Claim 2 which comprises means for administering ultrasound in combination with chemical enhancers and mechanical forces.

11. The apparatus of any one of Claims 2 to 10 wherein the molecules to be transported are drugs the patient is in need of.

**12.** The apparatus of any one of Claims 2 to 10 wherein the compound to be transported is an analyte to be measured.

**13.** The apparatus of any one of Claims 2 to 10 wherein the ultrasound is pulsed.

**14.** The apparatus of Claim 2 further comprising a composition for enhancing delivery of drugs across the skin comprising drug encapsulated in a liposome or polymeric carrier for application to the skin.

**15.** The apparatus of Claim 14 wherein the drug is in the form of a microparticle coated with a lipophilic or hydrophilic material enhancing transdermal penetration.

**16.** The apparatus of Claim 15 wherein the drug is a hydrophilic molecule coupled to a synthetic biodegradable polymer forming a microparticle.

**Patentansprüche**

**1.** Verfahren zur Extraktion eines Analyten, der in einer Blutprobe oder einer Probe einer Körperflüssigkeit gemessen werden soll, wobei das Verfahren das Applizieren einer wirksamen Ultraschallmenge auf eine für die Extraktion einer Probe geeignete Stelle und das Sammeln der extrahierten Probe umfasst, **dadurch gekennzeichnet, dass** für den Transport der Moleküle der Probe Ultraschall mit einer Frequenz zwischen 20 kHz und unter 1 MHz eingesetzt wird.

**2.** Apparat zur Verstärkung des transdermalen Transports von Molekülen, wobei der Apparat eine Vorrichtung zum Applizieren einer wirksamen Ultraschallmenge auf die Haut für den transdermalen Transport von Molekülen aufweist, **dadurch gekennzeichnet, dass** der Ultraschall für den Transport der Moleküle eine Frequenz von unter 2,5 MHz hat und der Apparat außerdem einen Verstärker aufweist, der aus der Gruppe ausgewählt ist, die besteht aus Kombinationen chemischer Verstärkermittel, die die Löslichkeit der Moleküle, die transportiert werden sollen, erhöhen, mit Agenzien, die die Fluidität von Lipid-Doppelschichten erhöhen, mit mechanischen Kraftfeldern, osmotischen Kraftfeldern, magnetischen Kraftfelder und elektrischen Kraftfeldern.

**3.** Apparat nach Anspruch 2, der eine Vorrichtung zur Verabreichung von Ultraschall mit einer Frequenz von 1 MHz oder darunter aufweist.

**4.** Apparat nach Anspruch 2 oder Anspruch 3, wobei der Verstärker Linolsäure in einer Ethanollösung ist.

**5.** Apparat nach Anspruch 2, der eine Vorrichtung zur Verabreichung von Ultraschall in Kombination mit einem elektrischen Kraftfeld aufweist, das aus der Gruppe ausgewählt ist, die aus einer Elektroporation und einer Iontophorese besteht.

**6.** Apparat nach Anspruch 5, wobei das elektrische Feld gepulst ist.

**7.** Apparat nach Anspruch 2, wobei der Apparat eine Vorrichtung für die Verabreichung von Ultraschall in Kombination mit einem magnetischen Kraftfeld aufweist.

**8.** Apparat nach Anspruch 2, wobei der Apparat eine Vorrichtung für die Verabreichung von Ultraschall in Kombination mit einem mechanischen Kraftfeld aufweist, dass durch Druck oder ein Vakuum erzeugt wird.

**9.** Apparat nach Anspruch 2, wobei der Apparat eine Vorrichtung für die Verabreichung von Ultraschall in Kombination mit einem osmotischen Kraftfeld aufweist.

**10.** Apparat nach Anspruch 2, wobei der Apparat eine Vorrichtung für die Verabreichung von Ultraschall in Kombination mit chemischen Verstärkern und mechanischen Kräften aufweist.

**11.** Apparat nach einem beliebigen der Ansprüche 2 bis 10, wobei die Moleküle, die transportiert werden sollen, Arzneimittel sind, die der Patient benötigt.

**12.** Apparat nach einem beliebigen der Ansprüche 2 bis 10, wobei die Verbindung, die transportiert werden soll, ein Analyt ist, der bestimmt werden soll.

**EP 0 781 150 B1**

**13.** Apparat nach einem beliebigen der Ansprüche 2 bis 10, wobei der Ultraschall gepulst ist.

**14.** Apparat nach Anspruch 2, wobei der Apparat außerdem eine Zusammensetzung zur Verstärkung der Zufuhr von Arzneimitteln durch die Haut aufweist, wobei die Zusammensetzung ein Arzneimittel aufweist, das für die Applikation auf die Haut in Liposomen oder einem polymeren Träger verkapselt ist.

**15.** Apparat nach Anspruch 14, wobei das Arzneimittel in Form eines Mikroteilchens vorliegt, das mit einem lipophilen oder hydrophilen Material beschichtet ist, das die transdermale Penetration verstärkt.

**16.** Apparat nach Anspruch 15, wobei das Arzneimittel ein hydrophiles Molekül ist, das an ein synthetisches biologisch abbaubares Polymer, das ein Mikroteilchen bildet, gekoppelt ist.


**Revendications**

**1.** Procédé destiné à extraire un analyte à mesurer dans un échantillon de sang ou de liquide organique comprenant les étapes consistant à appliquer à un site approprié à l'extraction d'un échantillon une quantité efficace d'ultrasons et à recueillir l'échantillon extrait, **caractérisé en ce que** l'on applique les ultrasons destinés à transporter les molécules de l'échantillon à une fréquence située entre 20 kHz et moins de 1 MHz.

**2.** Appareil destiné à améliorer le transport transdermique de molécules comprenant des moyens destinés à administrer à la peau une quantité efficace d'ultrasons destinés au transport transdermique de molécules, **caractérisé en ce que** les ultrasons destinés à transporter les molécules présentent une fréquence de moins de 2,5 MHz et l'appareil comprend en outre un amplificateur sélectionné parmi le groupe se composant de combinaisons d'amplificateurs chimiques d'agents améliorant la solubilité des molécules à transporter avec des agents améliorant la fluidité de bicouches lipidiques, des champs des forces mécaniques, des champs de forces osmotiques, des champs de forces magnétiques et des champs de forces électriques.

**3.** Appareil selon la revendication 2 qui comprend des moyens destinés à administrer des ultrasons à une fréquence de 1 MHz ou moins.

**4.** Appareil selon la revendication 2 ou la revendication 3 dans lequel l'amplificateur est de l'acide linoléique dans une solution d'éthanol.

**5.** Appareil selon la revendication 2 qui comprend des moyens destinés à administrer des ultrasons en combinaison avec un champ de forces électriques sélectionné parmi le groupe se composant de l'électroporation et de l'iontophorèse.

**6.** Appareil selon la revendication 5 dans lequel on pulse le champ électrique.

**7.** Appareil selon la revendication 2 qui comprend des moyens destinés à administrer des ultrasons en combinaison avec un champ de forces magnétiques.

**8.** Appareil selon la revendication 2 qui comprend des moyens destinés à administrer des ultrasons en combinaison avec un champ de forces mécaniques créé par pression ou vide.

**9.** Appareil selon la revendication 2 qui comprend des moyens destinés à administrer des ultrasons en combinaison avec un champ de forces osmotiques.

**10.** Appareil selon la revendication 2 qui comprend des moyens destinés à administrer des ultrasons en combinaison avec des amplificateurs chimiques et des forces mécaniques.

**11.** Appareil selon l'une quelconque des revendications 2 à 10 dans lequel les molécules à transporter sont des médicaments dont le patient a besoin.

**12.** Appareil selon l'une quelconque des revendications 2 à 10 dans lequel le composé à transporter est un analyte à mesurer.

**13.** Appareil selon l'une quelconque des revendications 2 à 10 dans lequel on pulse les ultrasons.

**14.** Appareil selon la revendication 2 comprenant en outre une composition destinée à améliorer la délivrance de médicaments à travers la peau comprenant du médicament encapsulé dans un liposome ou un support polymérique pour application à la peau.

**15.** Appareil selon la revendication 14 dans lequel le médicament est sous la forme d'une microparticule revêtue d'une matière lipophile ou hydrophile améliorant la pénétration transdermique.

**16.** Appareil selon la revendication 15 dans lequel le médicament est une molécule hydrophile couplée à un polymère biodégradable synthétique formant une microparticule.

FIG. 1a

FIG. 1b

FIG. 2

FIG. 3

FIG. 4

FIG. 5

FIG. 6a

FIG. 6b

FIG. 7a

FIG. 7b

FIG. 8

FIG. 9

FIG. 10

FIG. 11a

*FIG. 11b*

*FIG. 11c*

flux with and without us-exposure (1 MHz)

FIG. 12a

FIG. 12b

FIG. 13

FIG. 14